# EUROPEAN PATENT SPECIFICATION

(11) **EP 4 627 105 B1**
(45) Date of publication and mention of the grant of the patent: **10.06.2026**
(21) Application number: 23817418.9
(22) Date of filing: 01.12.2023
(51) Int. Cl.: C12Q 1/37, C40B 30/08, G01N 33/58, G01N 33/68

(54) **METHOD FOR IDENTIFYING A MODIFIED AMINO ACID DEGRON (MAAD)**
VERFAHREN ZUR IDENTIFIZIERUNG EINER MODIFIZIERTEN AMINOSÄURE DEGRON (MAAD)
PROCÉDÉ D'IDENTIFICATION D'UN DÉGRONS D'ACIDES AMINÉS MODIFIÉS (MAAD)

(30) Priority: 01.12.2022 EP 22210724; 15.03.2023 EP 23162141
(43) Date of publication of application: 08.10.2025
(73) Proprietor: ETH Zurich, 8092 Zurich (CH)
(72) Inventor: BODE, Jeffrey, 8003 Zürich (CH); HOFMANN, Raphael, Long Island City 11101 New York (US); CORN, Jacob, Ellery, 8057 Zürich (CH); MUHAR, Matthias, 8057 Zürich (CH); FARNUNG, Jakob, 44359 Dortmund (DE)
(74) Representative: Schaad, Balass, Menzl & Partner AG
(86) International application number: PCT/EP2023/083940
(87) International publication number: WO 2024/115746

(56) References cited:
- WO-A1-2019/089592
- WO-A1-2020/229818
- WO-A2-2022/221413
- VITTORIA ZOPPI ET AL: "Iterative Design and Optimization of Initially Inactive Proteolysis Targeting Chimeras (PROTACs) Identify VZ185 as a Potent, Fast, and Selective von Hippel Lindau (VHL) Based Dual Degrader Probe of BRD9 and BRD7", JOURNAL OF MEDICINAL CHEMISTRY, vol. 62, no. 2, 2018, pages 699 - 726, XP055564836
- A BRICELJ ET AL: "E3 Ligase Ligands in Successful PROTACs: An Overview of Syntheses and Linker Attachment Points", FRONTIERS IN CHEMISTRY, vol. 9, 2021, XP055828765
- LETO DARA E. ET AL: "Genome-wide CRISPR Analysis Identifies Substrate-Specific Conjugation Modules in ER-Associated Degradation", MOLECULAR CELL, vol. 73, no. 2, 2019, pages 377 - 389.e11, XP093051790
- ADAM T. MELVIN ET AL: "A Comparative Analysis of the Ubiquitination Kinetics of Multiple Degrons to Identify an Ideal Targeting Sequence for a Proteasome Reporter", PLOS ONE, vol. 8, no. 10, 2013, pages e78082, XP055510908
- VAN Q ET AL: "Transfecting Small Molecules, Peptides & Proteins for Pharmaceutical Research", 19 November 2010 (2010-11-19), https://www.technologynetworks.com/tn/posters/transfecting-small-molecules-peptides-230091, XP093052504, Retrieved from the Internet <URL:https://cdn.technologynetworks.com/ep/pdfs/transfecting-small-molecules-peptides.pdf> [retrieved on 20230607]

## Description

### Background of the invention

The present invention relates to a method for identifying a modified amino acid degron (MAAD).

Cellular protein homeostasis describes the essential process of regulating the function, localization and turnover of proteins. At the molecular level, this is often achieved by post-translational modifications that either directly control protein function or mark them for further processing by downstream effectors. While most well-studied post-translational modifications are deposited or erased by dedicated enzymes, amino acid side chains and the protein backbone itself can also experience a plethora of non-enzymatic modifications, such as oxidative damage or alkylation.

Selective protein degradation is typically initiated by a substrate receptor that recognizes its client protein via a characteristic sequence motif on the client, a so-called degron. The presence of a degron licenses the client for degradation by the general proteolytic machinery. Most prominently, ubiquitin ligases recognize a client degron and then modify the client via conjugation of the small protein tag ubiquitin onto lysine side chains. This is referred to as poly-ubiquitylation of a protein, which typically results in the recruitment and activation of the proteasomal complex for processive proteolysis. Specificity of this system is established by over 600 human ubiquitin ligases which can bind the specific degrons on their respective client proteins.

A degron can comprise unmodified or modified amino acid sequences or specific destabilizing terminal amino acids. Treatment with alkylating or oxidating agents can stimulate protein turnover, raising the possibility that individual chemical modifications can mark proteins for degradation.

WO2020229818A1 discloses a method of screening for peptides capable of binding to a ubiquitin protein ligase (E3), successful binding being determined by detecting the amount of a test protein in the cell.

WO2019007869A1 relates to a method of controlling the level of a polypeptide sequence comprising administering a polypeptide sequence fused to a ubiquitin targeting protein which comprises a minimal degron structural motif. Leto D. E. et a1: "Genome-wide CRISPR Analysis Identifies Substrate-Specific Conjugation Modules in ER-Associated Degradation", MOLECULAR CELL, vol. 73, no. 2, 2019, pages 377-389 discloses expression of GFP-degron fusion protein in a pool of mutant cells for identifying genes involved in targeted protein degradation.

However, the investigation of protein modifications is often hindered by the complexity and heterogeneity of the underlying mechanisms inducing degradation. Modifications can be added to large fractions of the proteome, but typically only affect a specific subset of each targeted protein. Each protein can also be subject to several independent modifications at multiple sites, greatly complicating functional interpretation. Elucidating the function of a protein modification is dramatically simpler with access to one or more specified proteins with one or more defined modifications. However, such modified proteins or peptides are often inaccessible by biological means. In addition, detection and enrichment of one specific modification from a sea of modified proteins is usually extremely difficult.

The knowledge of different modified amino acid degrons could be unique starting points for targeted degradation strategies (e.g. PROTACs). In contrast to sequence-based degrons, a modified amino acid degrons (MAAD) contains at least one non-natural amino acid and/or an amino acid or amino acid sequence, which is modified either by enzymatic modification, non-enzymatic modification or misincorporation of one or more amino acids. Therefore, the object of the present invention is to provide a screening method to identify modified amino acid degrons.

### Definitions

A **"modified amino acid degron"** or "MAAD" as used herein refers to a molecule comprising at least one non-natural or non-canonical amino acid and/or an amino acid or amino acid sequence, which is modified either by enzymatic modification, non-enzymatic modification or misincorporation of one or more amino acids. Modified amino acid degrons may be employed for targeted degradation strategies (e.g. PROTACs).

The term **"PROTAC"** as used herein refers to proteolysis-targeting chimeras. PROTAC generally have three components - an E3 ubiquitin ligase binding group (E3LB), a linker, and a protein binding group. PROTACs and PROTAC binding domains are known to the skilled person (see e.g. An et al, EBioMedicine. 2018 Oct; 36: 553-562).

The term **"reporter protein"** as used herein refers to any proteinaceous structure that can be measured and quantified by standard biochemical or optical methods. The reporter protein of the present disclosure is used to measure the turnover of the protein conjugate. Ideally, said reporter protein is not endogenously expressed or present in the cell incorporated, transduced or transfected with the protein conjugate. Preferred reporter proteins are Superfolder GFP (sfGFP) and mTagBFP2.

The terms **"organic moiety"** refers to a part of the **"organic molecule"** referred to, and in particular refers to a larger and characteristic part of organic molecules. Organic moieties are comprised in the protein conjugates of the present disclosure.

The terms **"functional end group"** refers to a functional group which is located at the very end of the organic molecule. In certain embodiments the functional end group comprises a heteroatom selected from the group consisting of oxygen, nitrogen and sulfur. In certain embodiments the functional group is selected from the group consisting of NH₂, SH₂, OMe and OEt. Preferably the functional group is NH₂ or OMe, most preferably NH₂.

The term **"incorporating"** as used in the context of the protein conjugate and the cells or cell lines of the present disclosure refers to a step in which the protein conjugate and the cells or the cell lines are incubated at conditions which allow the uptake of the protein conjugate into the cells. Such uptake may occur naturally. Such uptake may also be facilitated by appropriate means that are known to the person skilled in the art. One preferred means used to facilitate the uptake of the protein conjugates into the cells is electroporation.

The term **"measuring the turnover"** as used herein refers to the measurement of the abundance of the respective protein or protein conjugate over time, in particular its degradation rate. This measurement can be carried out, for example, using a Flurorescent In Cellulo Reporter (FLICR) screening assay. Flow cytometry allows quantification of intracellular reporter levels over time and therefore tracking of turnover.

The term **"control protein"** as used herein refers to a protein that is compared to the protein conjugate in terms of protein degradation. The control protein is typically not degraded or degraded to a significantly lower degree.

The term **"tagged [with]"** in the context of the present invention refers to the feature of a protein or protein conjugate of containing a group or moiety allowing to be identified by a cellular factor, and in particular refers to the tagging of a protein or protein conjugate with a modified amino acid degron (MAAD) to induce selective intracellular degradation.

### Embodiments of the invention

The present invention relates to the identification of novel modified amino acid degrons (MAADs) which are important for protein degradation.

The problem referred to above, *i.e.* to provide a screening method to identify modified amino acid degrons, is solved by the method according to claim 1. Further preferred embodiments are subject of dependent claims 2 to 13.

The method according to the present invention relates to method for identifying a modified amino acid degron (MAAD). It includes the following subsequent steps of
a. preparing an organic molecule of the general formula (I)

   X-T-Z (I)

   wherein
   T is an organic moiety comprising at least one canonical or non-canonical amino acid,
   X is a peptide comprising two or more canonical amino acids, said peptide being covalently linked to T by an amide bond; and
   Z is a functional end group comprising a heteroatom selected from the group consisting of oxygen, nitrogen and sulfur;
   with the proviso that if Z is OH, T does not consist of canonical amino acids;
b. preparing from the organic molecule of the general formula (I) a protein conjugate of the general formula (II)

   P-(L)ₚ-T-Z (II)

   wherein
   P is a reporter protein,
   T and Z have the same definition as in step a,
   L is a peptide comprising two or more canonical amino acids and p is 0 or 1;
c. incorporating the protein conjugate obtained in step b) in a cell line;
**d.** measuring the turnover of the protein conjugate and comparing it with the turnover of a control protein to identify if the protein conjugate is tagged with a MAAD inducing intracellular protein degradation,
**e.** generating a pool of mutant cells, each defective in a different gene, and
f. incorporating the MAAD-tagged protein conjugate identified in step d) into the pool of mutant cells generated in step e) to isolate mutant cells that fail to selectively degrade the protein conjugate, thus identifying factors mediating the selective degradation of the MAAD-tagged protein conjugate.

As mentioned above, the term "modified amino acid" encompasses a non-natural or non-canonical amino acid as well as an amino acid or amino acid sequence, which is modified either by enzymatic modification, non-enzymatic modification or misincorporation of one or more amino acids. In the context of the present invention, the amino acid is modified due to being non-canonical, in which case Z can be OH, or due to being modified by Z being other than OH, in which case the amino acid can be canonical. Specifically, an amino acid or amino acid sequence having a C-terminus modified by amidation falls under the definition of a modified amino acid.

By combining synthetic organic chemistry, biochemistry, genetics and cell biology, the screening method according to the present invention allows a fast method for the identification of modified amino acid degrons. Specifically, the present invention further allows to identify the factors mediating the selective degradation of a protein tagged with the MAAD in a straightforward manner. Ultimately, the method according to the present invention provides a general, high-throughput workflow for evaluating the effects of modified amino acids on protein stability in cells. In addition, it allows access to new drug molecules, in particular new PROTACS.

As mentioned above, the first step of the screening method according to the present invention relates to the preparation of an organic molecule of the general formula (I)

X-T-Z (I)

wherein T represents an organic moiety comprising at least one canonical or non-canonical amino acid.. X comprises two or more canonical amino acids.

Z is a functional end group comprising a heteroatom selected from the group consisting of oxygen, nitrogen and sulfur. Examples of such functional end groups are OH, OMe, OEt, NH₂ and SH₂.

If in the compound of formula (I) Z is OH, T does not consist of canonical amino acids. In this case, it may be a peptide comprising or consisting of non-canonical amino acids. In other words, the definition of compound A covers both compounds, in which T comprises one or a sequence of non-canonical amino acids, as well as compounds, in which T consists of one or a sequence of canonical amino acids, the carboxy group of the C-terminal amino acid being modified, in particular amidated.

In a second step b), a protein conjugate of the general formula (II)

P-(L)ₚ-T-Z (II)

wherein
P is a reporter protein,
T and Z have the same definition as in step 1
L is a peptide comprising two or more canonical amino acids and p is 0 or 1;
is prepared. Thus, the protein conjugate is a reporter protein that carries a protein modification obtained in step a).

According to a preferred embodiment of the present invention, the reporter protein is selected from the group consisting of a fluorescent protein, green fluorescence protein (GFP), a variant of GFP, preferably green fluorescence protein.

Preferably, the preparation of the protein conjugate of step b) is performed by chemoenzymatically conjugating the compound of step a) to the reporter protein. For example, the conjugating enzyme sortase can be used to attach the organic molecule of formula I to the reporter protein P. Sortase catalyzes the mild and selective reaction of a C-terminal recognition motif with an N-terminal motif. In some aspects of the invention, the C-terminal sortase recognition motif is a sortase A (SrtA) recognition motif. In some aspects of the invention, the sortase A recognition motif A-MO1 is LPXTG (SEQ ID NO: 1), wherein X is any amino acid. In some aspects of the invention, the sortase A recognition motif A-MO2 is LPETGG (SEQ ID NO: 2). In some aspects of the invention, the C-terminal sortase recognition motif is a sortase B recognition motif. In some aspects of the invention, the sortase B recognition motif B-MO1 is NPQTN (SEQ ID NO: 3 or B-MO2 NPKTG (SEQ ID NO: 4).

In some aspects of the invention, the linker comprises at least one glycine-serine repeat. In some aspects of all the embodiments of the invention, the linker comprises 3 glycine-serine (GS-MO: SEQ ID NO: 5) or 4 glycine-serine (GS-MO2:SEQ ID NO: 6) repeats.

In some aspects of all the embodiments of the invention, the N-terminal sortase recognition motif consists of more than two glycine residues. In some aspects of all the embodiments of the invention, the N-terminal sortase recognition motif consists of 3-10 glycine residues (e.g. GGG). In some aspects of all the embodiments of the invention, the N-terminal sortase recognition motif consists of five glycine residues GGGGG (G-MO1: SEQ ID NO: 7).

Most preferably, sortase A catalyzes the reaction of the C-terminal recognition motif LPETGG (A-MO2: SEQ NO. 2) with an N-terminal recognition motif consisting of 3 glycine residues GGG.

| Name | SEQ ID NO.: | Sequence |
|---|---|---|
| sortase recognition motif | | |
| A-MO1 | 1 | LPXTG |
| A-MO2 | 2 | LPETGG |
| B-MO1 | 3 | NPQTN |
| B-MO2 | 4 | NPKTG |
| GS-MO | 5 | GSGSGS |
| MS-Mo2 | 6 | GSGSGSGS |
| G-MO1 | 7 | GGGGG |

In the next step of the method according to the present invention, the protein conjugate obtained in step b) is incorporated in a cell line, preferably an immortalized mammalian cell line. This can for example be done via electroporation. Preferred examples of the cell line include human erythroleukemia cell line K562 as a highly scalable model and human embryonic kidney derived 293T cells as a non-cancerous cell context.

Upon incorporation, the turnover of the protein conjugate is measured and compared with the turnover of a control protein to identify if the protein conjugate is tagged with a MAAD inducing intracellular protein degradation. This can be done, for example, with a Flurorescent In Cellulo Reporter (FLICR) screening assay. Flow cytometry allows quantification of intracellular reporter levels over time and therefore tracking of turnover. The control experiment can be carried out with a reporter protein, that carries a C-terminal RXXGXX motif, previously identified to induce proteasomal protein turnover (Koren, I.; Timms, R. T.; Kula, T.; Xu, Q. ; Li, M. Z. ; Elledge, S. J., The Eukaryotic Proteome Is Shaped by E3 Ubiquitin Ligases Targeting C-Terminal Degrons. Cell 2018, 173 (7), 1622-1635 e14). Specifically, sfGFP (superfolder green fluorescent protein) carrying this degron (Pep2-RXXGXX: SEQ ID No. 17) scored strongly in the FLICR assay. Any modification that induces loss of fluorescent signal by at least two-fold relative to non-modified control within 8h, is considered to be a protein conjugate that induces degradation.

Additionally, the cells can be treated with inhibitors of the proteasome (epoxomicin) or ubiquitylation (TAK243) to prevent turnover of degron-conjugated sfGFP, thus elucidating the cellular pathways underlying selective protein degradation by means of the FLICR assay.

According to the invention, the protein conjugate tagged with the MAAD in step d) is screened for the genome-wide identification of the genes involved in selective degradation of the protein, thus elucidating the cellular machinery underlying the recognition and removal of the degron.

Thus, the method of the present invention comprises after step d) the further subsequent steps of
e) generating a pool of mutant cells, each defective in a different gene, and
f) incorporating the MAAD-tagged protein conjugate identified in step d) into the pool of mutant cells generated in step e) to isolate mutant cells that fail to selectively degrade the protein conjugate, thus identifying factors mediating the selective degradation of the MAAD-tagged protein conjugate.

Regarding step e), this is according to a preferred embodiment performed using a CRISPR-based inducible knockout system, in particular using a doxycycline-dependent knockout system, as pointed out in detail in the context of the working examples. Specifically, this includes the engineering of K562 erythroleukemia cells to express the Cas9 nuclease under a doxycycline-inducible promotor. Using this screening system, addition of single guide RNA (sgRNA) directs Cas9 to disrupt any target locus, but only upon addition of doxycycline, thus allowing to rapidly perturb and study cellular pathways, including those necessary for cell survival.

Regarding step f), the isolation of mutant cells is preferably performed using a Fluorescent In Cellulo Reporter (FLICR) screening assay, as will also be explained in detail in the context of the working examples.

Preferably, step f) involves simultaneously co-delivering a reporter protein different to the one of step b) and being conjugated to a canonical degron sequence, in particular a RXXGXX degron sequence, thus allowing to isolate mutants that are still capable of degrading a protein conjugate containing a sequence-based degron but incapable of degrading a protein conjugate containing a MAAD.

According to a specific way of performing steps e) and f), cells are transduced with a pool of lentiviral vectors that each carry a different sgRNA targeting one of more than 18'000 protein coding genes with four different guide RNAs targeting each gene. Following induction of Cas9 expression, a first reporter protein (for example sfGFP) tagged with a modified amino acid degron (MAAD-tagged) is incorporated into the cell pool via electroporation alongside a second reporter protein, for example mTagBFP2-SRT-His6 (SEQ ID NO. 20), tagged with the unrelated degron sequence RXXGXX. Following the onset of cellular protein degradation, fluorescent activated cell sorting (FACS) is then performed to isolate mutant cells that have degraded RXXGXX-tagged mTagBFP2 (mTagBFP2-Pep2-RXXGXX, SEQ ID. No. 27), but failed to degrade the protein conjugate of the general formula (II). Thus, mutant cells can be detected which exhibit a specific defect in MAAD clearance but have a normally functioning ubiquitin-proteasome system. Based thereon, sgRNA that are enriched in MAAD-deficient cells are then identified by deep sequencing. Ultimately, the genes encoding for factors mediating the selective degradation of the MAAD-tagged protein can thereby be identified.

With the method according to the present invention it was shown that amidation of a protein's C-terminus, i.e. a compound of formula IIa,

P-(L)ₚ-T-NH₂ (IIa),

wherein S is a canonical amino acid, is sufficient to mark proteins for selective degradation via the SCF^{FBXO31} complex. Thus, a minimal chemical modification was found to be sufficient to obtain an excellent amino acid degron.

In one embodiment of the present invention wherein T is an organic moiety of the general formula (III)

S-(Y)ₘ (III)

wherein S is an organic moiety of the general formula 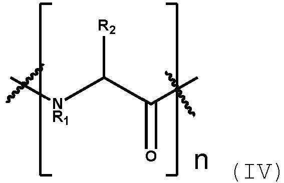 wherein
R₁ is hydrogen, a linear or branched, saturated or unsaturated C₁ to C₁₀ alkyl residue, or forms together with R₂ a ring system
R₂ is selected from the group consisting of hydrogen, substituted or unsubstituted alkyl, substituted or unsubstituted alkenyl, substituted or unsubstituted alkynyl, substituted or unsubstituted cycloalkyl, substituted or unsubstituted het-erocycloalkyl, substituted or unsubstituted aryl, substituted or unsubstituted heteroaryl, substituted or unsubstituted aralkyl, substituted or unsubstituted aralkenyl, substituted or unsubstituted heteroaralkyl, and substituted or unsubstituted heteroaralkenyl,
n is 1 to 5,
Y is a canonical amino acid or a peptide comprising two or more canonical amino acids and m is 0 or 1.

S can be essentially any small organic molecule that has a protein backbone which allows for N- and C- terminal binding to X and Y or Z. Thus, the method according to the present invention allows the screening of libraries of organic molecules to find modified amino acid degrons that induce selective degradation, i.e., the degradation is initiated by a specific substrate receptor.

Within the context of the present invention "alkyl" means a straight or branched chain unsubstituted hydrocarbon group, preferably comprising 1 to 20 carbon atoms.

The term heteroalkyl refers to an alkyl, alkenyl or alkynyl group as defined herein, where one or more and preferably 1, 2 or 3 carbon atoms are replaced independently of each other by an oxygen, nitrogen, phosphorous or sulphur atom, for example an alkoxy group containing from 1 to 10 carbon atoms, preferably from 1 to 6 carbon atoms, e.g. 1 to 4 carbon atoms, such as methoxy, ethoxy, propoxy, iso-propoxy, butoxy or tert.-butoxy; a (1-4 C)alkoxy(1-4C)alkyl group such as methoxymethyl, ethoxymethyl, 1-methoxyethyl, 1-ethoxyethyl, 2-methoxyethyl or 2-ethoxyethyl; or a cyano group; or a 2,3-dioxyethyl group.

Within the context of the present invention, "substituted alkyl" means an alkyl group substituted with one to four substituents selected from the group consisting of fluoro, chloro, bromo, iodo, trifluoromethyl, trifluoromethoxy, hydroxy, alkoxy, cycloalkoxy, heterocyclooxy, oxo, alkanoyl, aryloxy, alkanoyloxy, amino, alkylamino, arylamino, aralkylamino, cycloalkylamino, heterocycloamino, disubstituted amines in which the 2 amino substituents are selected from alkyl, aryl or aralkyl, alkanoylamino, aroylamino, aralkanoylamino, substituted alkanoylamino, substituted arylamino, substituted aralkanoylamino, thiol, alkylthio, arylthio, aralkylthio, cycloalkylthio, heterocyclothio, alkylthiono, arylthiono, aralkylthiono, alkylsulfonyl, arylsulfonyl, aralkylsulfonyl, -SO₂NH₂, substituted sulfonamido, nitro, cyano, carboxy, -CHO, -CH(COOH)₂, CH (CONH₂)₂, - CH(COOalkyl)₂, -CONH₂, -CONHalkyl, -CONHaryl, CONHaralkyl, - NHCOalkyl, -NHCOaryl, -NHCOaralkyl, alkoxycarbonyl, guanidine. Preferably, a substituted alkyl is selected from the group consisting of a linear alkyl substituted by hydroxy, CHO, carboxy, CH(COOH)₂, -NHCOalkyl, mercapto, imidazolyl, methylthio, aryl, amino, guanidine, CHO, and -CH(COOH)₂. Within the context of the present invention, "alkenyl" means a straight or branched chain unsubstituted hydrocarbon group which contains at least one double bond, preferably comprising 1 to 20 carbon atoms.

Within the context of the present invention "substituted alkenyl" means an alkenyl group substituted with one to four substituents. The substituents include one to four substituents as recited above as alkyl substituents.

Within the context of the present invention "alkynyl" means a straight or branched chain unsubstituted hydrocarbon group which contains at least one triple bond, preferably comprising 1 to 20 carbon atoms.

Within the context of the present invention "substituted alkynyl" means an alkynyl group substituted with one to four substituents. The substituents include one to four substituents as recited above as alkyl substituents.

Within the context of the present invention, "cycloalkyl" means an optionally substituted, saturated cyclic hydrocarbon ring systems containing 1 to 3 rings and 3 to 7 carbons per ring which may be further fused with one or more heterocyc-loalkyl, aryl or heteroaryl groups, wherein when substituted, the substituents will include one or more substituents as recited above as alkyl substituents.

Within the context of the present invention, "aryl" means a monocyclic or bicyclic aromatic hydrocarbon group having 6 to 12 carbon atoms in the ring portion.

Within the context of the present invention, "substituted aryl" means an aryl group substituted by one to four substituents selected from alkyl, substituted alkyl, halo, trifluoromethoxy, trifluoromethyl, hydroxy, alkoxy, cycloal-kyloxy, heterocyclooxy, alkanoyl, alkanoyloxy, amino, alkylamino, aralkylamino, cycloalkylamino, heterocycloamino, dialkylamino, alkanoylamino, thiol, alkylthio, cycloalkylthio, heterocyclothio, ureido, nitro, cyano, carboxy, carboxyalkyl, carbamyl, alkoxycarbonyl, alkylthiono, arylthiono, alkysulfonyl, sulfonamido, and aryloxy.

Within the context of the present invention, "heterocyclo" means an optionally substituted, fully saturated or unsaturated, aromatic or nonaromatic cyclic group, which is a 4 to 7 membered monocyclic, 7 to 11 membered bicyclic, or 10 to 15 membered tricyclic ring system, having at least one heteroatom in at least one carbon atom-containing ring, each ring of the heterocyclic group containing a heteroatom may have 1, 2 or 3 heteroatoms, wherein the term "heteroatoms" shall include oxygen, sulfur and nitrogen; and wherein when substituted, the substituted heterocyclo group will include one or more substituents as recited above as alkyl substituents, preferably hydroxy, alkylhydroxy, amino, nitro, fluoro, chloro, bromo, iodo and CHO.

Within the context of the present invention, "aralkyl" means a radical -RₐR_{b} where Rₐ is an alkylene group and R_{b} is an aryl group as defined herein, e.g., benzyl, phenylethyl and the like.

Within the context of the present invention "aralkenyl" means a radical -RₐR_{b} where Rₐ is an alkenylene group and R_{b} is an aryl group as defined herein, e.g., 3-phenyl-2-propenyl, and the like.

Within the context of the present invention, "heteroaralkyl" means an alkyl group substituted with a heterocyclic ring as defined above.

Within the context of the present invention, "heteroaralkenyl" means an alkenyl group substituted with a heterocyclic ring as defined above.

In the compound of formula (I), X is a peptide comprising two or more canonical amino acids and can comprise at least a part of a C-terminal motif which allows to link the reporter protein to the organic molecule by means of an enzyme.

Y is a canonical amino acid or a peptide comprising two or more canonical amino acids and may be present or not. If m is 0, end group Z is directly bound to the carbonyl group of S, thereby forming an amide, an carbonic acid, an ester or a thioester. If m is 1, Y is preferably a canonical amino acid or a peptide comprising from two to five amino acids.

In one embodiment of the present invention n is 1 or 2, preferably 1. According to this embodiment, S thus comprises 1 or 2 peptide units in its backbone.

In a further embodiment of the present invention R₂ is selected from the group consisting of substituted alkyl, substituted or unsubstituted alkenyl, substituted aryl, substituted or unsubstituted heteroaryl, substituted or unsubstituted aralkyl, substituted or unsubstituted aralkenyl, substituted or unsubstituted heteroaralkyl, and substituted or unsubstituted heteroaralkenyl, preferably substituted alkyl and substituted aralkyl.

In a preferred embodiment S is a modified amino acid. Examples of modified amino acids are methylated lysine, oxidized tyrosine, oxidized tryptophan or oxidized methionine. Within the context of the present invention, the term "modified amino acid" means an amino acid that is different from a canonical amino acid.

In a preferred embodiment, S comprises a non-canonical d-amino acid or is a non-canonical D-amino acid.

In one embodiment of the present invention, S is an enzymatically modified amino acid. This has the advantage that S is easily accessible.

In one embodiment of the present invention, S is a chemically modified amino acid which allows to provide modifications that are not accessible by enzymatic means

In one embodiment of the present invention, Z is selected from the group consisting of NH₂, SH₂, OMe and OEt, preferably NH₂ and OMe, most preferably NH₂. Preferably S has a C terminal modification, i.e. m is 0, most preferably selected from the group consisting of an amide, a methyl ester and an ethyl ester. In this case, S is preferably a canonical amino acid.

As already mentioned before, amidation of a protein's C-terminus, is sufficient to mark proteins for selective degradation via the SCF^{FBXO31} complex.

In a preferred embodiment of the present invention, S is selected from the group consisting of

| | |
|---|---|
| 1 | 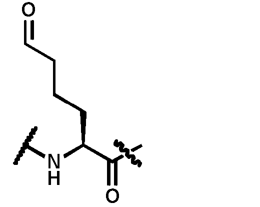 |
| 2 | 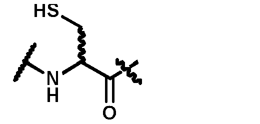 |
| 3 | 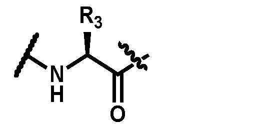 wherein R₃ is the residue of a canonical amino acid |
| 4 | 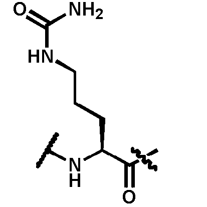 |
| 5 | 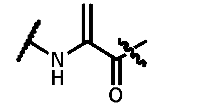 |
| 6 | 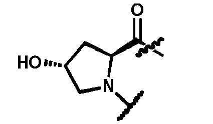 |
| 7 | 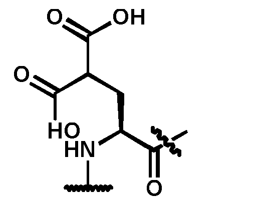 |
| 8 | 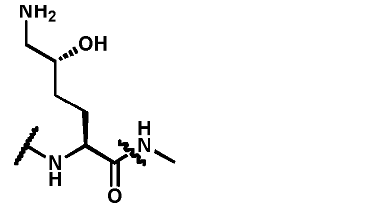 |
| 9 | 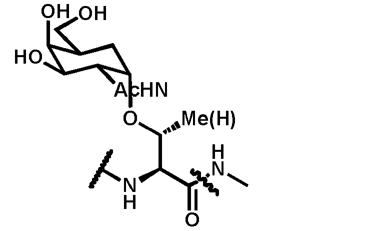 |
| 10 | 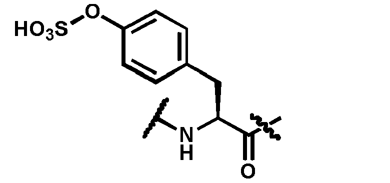 |
| 11 | 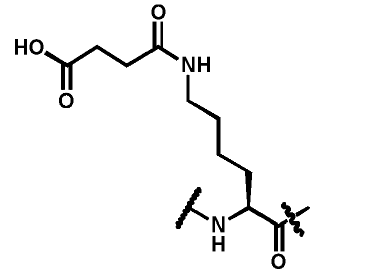 |
| 12 | 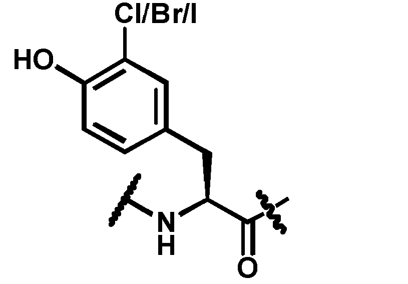 |
| 13 | 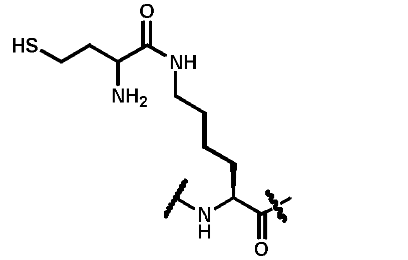 |
| 14 | 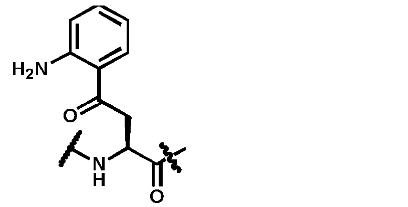 |
| 15 | 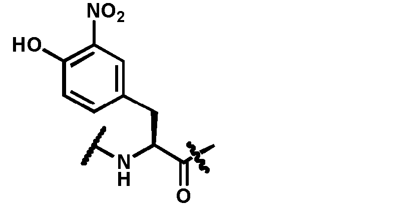 |
| 16 | 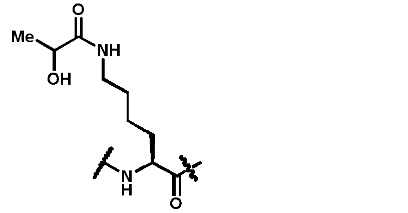 |
| 17 | 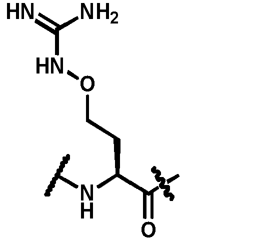 |
| 18 | 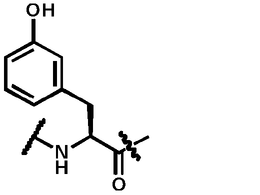 |
| 19 | 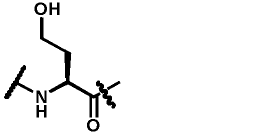 |
| 20 | 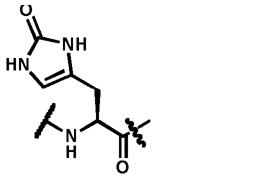 |
| 21 | 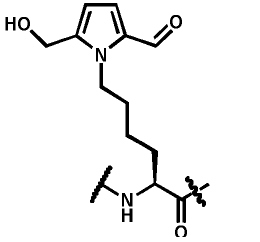 |
| 22 | 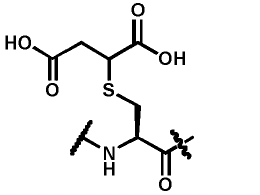 |
| 23 | 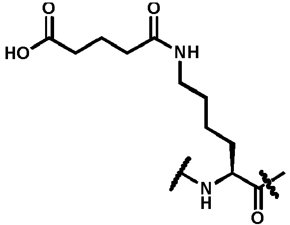 |

In one embodiment of the present invention, the protein turnover, i.e., the degradation rate of the protein is measured by a fluorescent *in cellulo* reporter screening assay, that is, the turnover is measured in isolated live cells.

Preferably, the assay is carried out in a human cell line, most preferably in the human erythroleukemia cell line K562. These cells provide an ideal platform, since their suspension growth allows parallel handling, sampling of multiple time points without disrupting cultures and they are well established models for CRISPR engineering and protein turnover studies in the lab.

To establish MAAD function of the initial screen candidates, it can be tested whether modifications induce degradation in a second protein context that is distinct in both sequence and structure.

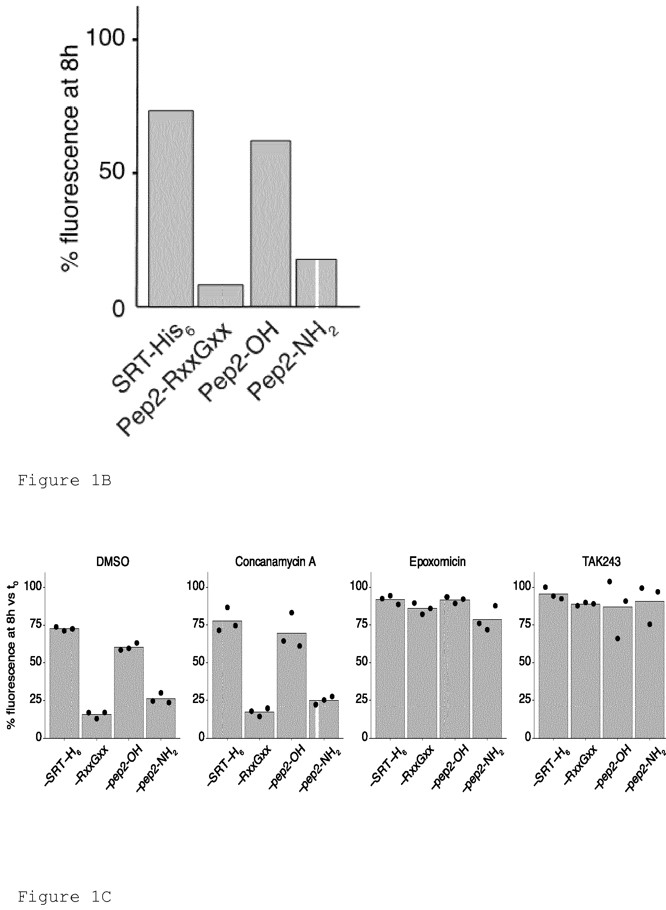

For example, compounds of formula III carrying a primary C-terminal amide (i.e. with Z being NH₂) were also efficiently degraded when delivered to human embryonic kidney-derived HEK293T cells (Fig.1B) establishing that C-terminal amidation induces protein degradation in different amino acid-, protein- and cell contexts. This allows to find out whether MAAD candidates act ubiquitously or in a tissue-dependent manner.

Since the fluorescent readout of the FLICR assay can also report protein unfolding or fluorophore quenching, the hits can optionally be further validated by electroporation followed by immunoblotting time course.

Moreover, to ensure that candidate MAADs are actively removed via core proteolytic pathways, they can be tested by inhibition of the proteasome (epoxomicin) or lysosomal acidification (concanamycin A).

As mentioned above, steps a) to d) of the method of the present invention form the basis for additionally identifying factors mediating the selective degradation of a protein tagged with the MAAD by performing additional steps e) and f) described above.

As also mentioned above, the genome-wide identification of the genes involved in the selective intracellular degradation of the MAAD-tagged protein conjugate can comprise a CRISPR system to induce knockout and profile cells without prolonged culture. In the context of this application, the term "CRISPR system" includes any system involving a Cas protein variant, in particular CasMini or Cas-CLOVER, as well as CRIPSR interference or base-editing methods. Alternatively, a RNA-interference method (as described in Berns, K., Hijmans, E.M., Mullenders, J., Brummelkamp, T.R., Velds, A., Heimerikx, M., Kerkhoven, R.M., Madiredjo, M., Nijkamp, W., Weigelt, B. and Agami, R., 2004. A large-scale RNAi screen in human cells identifies new components of the p53 pathway. Nature, 428(6981), pp.431-437) or a gene-trap mutagenesis method (as described in Carette, J.E., Guimaraes, C.P., Varadarajan, M., Park, A.S., Wuethrich, I., Godarova, A., Kotecki, M., Cochran, B.H., Spooner, E., Ploegh, H.L. and Brummelkamp, T.R., 2009. Haploid genetic screens in human cells identify host factors used by pathogens. Science, 326(5957), pp.1231-1235) may be used.

In one embodiment, the vector is a viral vector. The viral gene delivery system may be an RNA-based or DNA-based viral vector. Viral vectors include retroviral vectors, lentiviral vectors (e.g., derived from HIV-1, HIV-2, SIV, BIV, FIV etc.), gammaretroviral vectors, adenoviral (Ad) vectors (including replication competent, replication deficient and gutless forms thereof), adeno-associated virus-derived (AAV) vectors, simian virus 40 (SV-40) vectors, bovine papilloma virus vectors, Epstein-Barr virus vectors, herpes virus vectors, vaccinia virus vectors, Harvey murine sarcoma virus vectors, murine mammary tumour virus vectors, Rous sarcoma virus vectors and Sendai virus vectors. In a further embodiment, the viral vector is selected from: a lentiviral vector, an adeno-associated virus vector or a Sendai virus vector. In a yet further embodiment, the viral vector is a lentiviral vector. In the method according to the present invention preferably a pool of lentiviral vectors is used. Lentiviral vectors are well known in the art. Lentiviral vectors are complex retroviruses capable of integrating randomly into the host cell genome, which, in addition to the common retroviral genes gag, pol, and env, contain other genes with regulatory or structural function (e.g., accessory genes Vif, Nef, Vpu, Vpr). Lentiviral vectors have the advantage of being able to infect non-dividing cells and can be used for both in vivo and ex vivo gene transfer and expression of nucleic acid sequences. For example, recombinant lentiviral vector capable of infecting a non-dividing cell wherein a suitable host cell is transfected with two or more vectors carrying the packaging functions, namely gag, pol and env, as well as rev and tat. In one embodiment a library of more than 70'000 sgRNA vectors that target 18,053 protein coding genes with 4 independent sgRNAs are screened, as mentioned above.

In one embodiment of the present invention a pool of mutant cells is generated by performing a modified FLICR assay, as mentioned above. As also pointed out above, cells are transduced with a pool of lentiviral vectors that each carry a different sgRNA targeting one of >18,000 protein coding genes (with 4 different guide RNAs targeting each gene). Following induction of Cas9 expression, compound of formula III is delivered to cells via electroporation alongside a second reporter protein which carries the unrelated degron sequence (Pep2-RXXGXX; SEQ ID No. 17). Following the onset of cellular protein degradation, fluorescence activated cell sorting (FACS) can be performed to isolate mutant cells that have degraded said second reporter protein, but failed to degrade the compound of formula III. These mutant cells thus show a specific defect in MAAD clearance but have a normally functioning ubiquitin-proteasome system.

In a further embodiment, deep sequencing is carried out, as also pointed out above. This allows to identify sgRNAs that are enriched in MAAD-deficient cells and therefore likely target a specific MAAD receptor or critical downstream effectors.

### EXAMPLES

### General materials and methods

### Reagents and solvents

Fmoc-amino acids with suitable side-chain protecting groups, HATU (1-[bis(dimethylamino)methylene]-1H-1,2,3-triazolo[4,5-b]pyridinium 3-oxide hexafluorophosphate) were purchased from Peptides International (Louisville, KY, USA), ChemImpex (Wood Dale, IL, USA) and Merck Milipore. HPLC grade CH3CN from Sigma-Aldrich was used for analytical and preparative HPLC purification. Trifluoroacetic acid for HPLC analytical and preparative HPLC purification was purchased from ABCR. DMF (> 99.8%) from Sigma-Aldrich and N-methylpyrrolidine from ABCR were directly used without further purification for solid phase peptide synthesis. Other commercially available reagents and solvents were purchased from Sigma-Aldrich (Buchs, Switzerland), Acros Organics (Geel, Belgium) and TCI Europe (Zwijndrecht, Belgium).

### Characterization

High-resolution mass spectra were recorded by the Molecular and Biomolecular Analysis Service (MoBiAS) at ETH Zurich with a Bruker maXis instrument (ESI-MS measurements) equipped with an ESI source and a Q-TOF detector. Reaction monitoring was performed on a Bruker microFLEX instrument (MALDI-TOF) using 4-hydroxy-α-cyanocinnamic acid as matrix.

### Purification

Peptides were analyzed and purified by reverse phase high performance liquid chromatography (RP-HPLC) on JASCO analytical and preparative instruments equipped with dial pump, mixed and in-line degasser, a variable wavelength UV detector (simultaneous detection of the eluent at 220 nm, 254 nm and 301 nm) and a Rheodyne injector with a 200 µL or 10 mL injection loop. Columns were heated to 60 °C using a Jetstream 2 column heater (analytical) or a H₂O water bath (preparative). The mobile-phase for RP-HPLC was Milipore-H2O containing 0.1% (v/v) TFA and HPLC grade CH3N containing 0.1% (v/v) TFA. Analytical HPLC was performed on Shiseido Capcell Pak C18 (5 µm, 4.6 mm I.D. x 250 mm) columns at a flow rate of 1 mL/min. Preparative HPLC was performed on Shiseido Capcell Pak MGIII (5 µm, 20 mm I.D. x 250 mm) at a flow rate of 10 mL/min.

### General analytical HPLC methods:

flow 1 mL/min, isocratic 10% CH3CN for 3 min, then gradient from 10% to 95% CH₃CN in 14 min.

### General preparative HPLC methods:

flow 10 mL/min, isocratic 10% CH3CN for 5 min, then gradient from 10% to 65% CH₃CN in 28 min.

### Solid phase peptide synthesis

Loading of amino acids on solid support was performed as followed:
**Chloro-trityl resin:** The amino acid (1.20 equiv of desired loading) was dissolved in CH2Cl2 (200 mM). NMM (2 equiv) was added to the solution. The solution was given to preswollen chloro-trityl resin and shaken for 1h. The resin was washed with CH2Cl2 and DMF. Remaining chloro-trityl moieties were capped with CH2Cl2/MeOH/NMM (17:2:1, v:v:v) for 1 min. The capping step was repeated once. The resin was washed with CH₂Cl₂ and DMF. The resin was dried using a N2 stream prior to usage.

**Rink amide resin:** Fmoc-Rink amide resin was deprotected using 20 vol% Piperidine in DMF for 2x5 min. The resin was washed thoroughly. Amino acid (1.2 equiv of desired loading) and HCTU (0.95 equiv of amino acid) were dissolved in DMF (200 mM). NMM (2 equiv of amino acid) was added. The solution was added to the preswollen Rink amide resin and shaken for 18 h. The resin was washed with CH2Cl2 and DMF. The resin was dried using a N2 stream prior to usage.

Peptides were synthesized on a Multisyntech Syro I parallel synthesizer using Fmoc-SPPS chemistry.

**General methods on Multisyntech Syro I parallel synthesizer:** Amino acids were dissolved in DMF to a concentration of 0.5 M. HATU was dissolved in DMF to a concentration of 0.5 M. DIPEA was dissolved in NMP to a concentration of 2 M. Amino acid, HATU and DIPEA are mixed to a final concentration of 0.2 M, 0.2 M and 0.4 M, respectively, and added to the resin. The resin was agitated for 45 min. Coupling steps were repeated once.

Capping was performed with acetic anhydride. 20 vol% acetic anhydride in DMF was mixed with 2 M DIPEA at a ratio of 3:2 and added to the resin. The resin was agitated for 5 min. The capping step was repeated once.

Fmoc deprotection was performed with 20 vol% piperidine in DMF for 10 min. The deprotection step was repeated once.

### Solid Phase Peptide Synthesis

For fluorescein modified peptides, Boc-Lys(Fmoc)-OH (2 equiv) was coupled as the N-terminal residue using HATU. Fmoc was removed by treatment with 20 vol% piperidine. All following steps were performed in the dark. FITC (3 equiv) and NMM (6 equiv) were dissolved in DMF, given to the resin and shaken for 2 h. The resin was thoroughly washed using DCM and DMF. Peptide was cleaved form the resin using TFA/DODT/H2O (95:2.5:2.5, v/v) for 1 h. The resin was removed by filtration and the filtrate concentrated under reduced pressure. The solution was triturated with Et₂O and centrifuged to obtain crude peptide. The crude peptide was dissolved in H₂O/CH3N (1:1, v/v) + 0.1% (v/v) TFA and purified using preparative HPLC.

The following peptides were synthesized:

| Name | SEQ ID NO. : | Sequence |
|---|---|---|
| **Probes for sortase conjugation** | | |
| Pep1-DOPA | 8 | GGGKDLEGKGGS**X**GSGS**X**GGSKYPYDVPDYAKD-[OH] |
| Pep1-HNE | 9 | GGGKDLEGKGGS**X**GSGS**X**GGSKYPYDVPDYAKD-[OH] |
| Pep1-CML | 10 | GGGKDLEGKGGS**X**GSGS**X**GGSKYPYDVPDYAKD-[OH] |
| Pep1-hCit | 11 | GGGKDLEGKGGS**X**GSGS**X**GGSKYPYDVPDYAKD-[OH] |
| Pep1-Ala | 12 | GGGKDLEGKGGS**A**GSGS**A**GGSKYPYDVPDYAKD- [OH] |
| Pep1-Tyr | 13 | GGGKDLEGKGGS**Y**GSGS**Y**GGSKYPYDVPDYAKD-[OH] |
| Pep1-NH2 | 14 | GGGKDLEGKGGS**A**GSGS**A**GGSKYPYDVPDYAKD-[NH₂] |
| Pep1-Ser-OH | 15 | GGGKDLEGKGGSAGSGSAGGSKYPYDVPDYAKS-[OH] |
| Pep1-Ser-NH2 | 16 | GGGKDLEGKGGSAGSGSAGGSKYPYDVPDYAKS-[NH2] |
| Pep2-RXXGXX | 17 | GGGRRLEGKEEDEKGSRASDRFRGLR-[OH] |
| Pep2-OH | 18 | GGGRRLEGKEEDEKGSRASDDFRDLR-[OH] |
| Pep2-NH2 | 19 | GGGRRLEGKEEDEKGSRASDDFRDLR-[NH2] |
| | | |
| | | |

| Fluorescent proteins | | |
|---|---|---|
| mTagBFP 2-SRT-His6 | 20 | MVSKGEELIKENMHMKLYMEGTVDNHHFKCTSEGEGKPYEGTQTM RIKVVEGGPLPFAFDILATSFLYGSKTFINHTQGIPDFFKQSFPE GFTWERVTTYEDGGVLTATQDTSLQDGCLIYNVKIRGVNFTSNGP VMQKKTLGWEAFTETLYPADGGLEGRNDMALKLVGGSHLIANAKT TYRSKKPAKNLKMPGVYYVDYRLERIKEANNETYVEQHEVAVARY CDLPSKLGHKLNSGGLPETGGHHHHHHV |
| sfGFP-SRT-His6 | 21 | MSMSKGEELFTGVVPILVELDGDVNGHKFSVRGEGEGDATNGKLT LKFICTTGKLPVPWPTLVTTLTYGVQCFARYPDHMKQHDFFKSAM PEGYVQERTISFKDDGTYKTRAEVKFEGDTLVNRIELKGIDFKED GNILGHKLEYNFNSHNVYITADKQKNGIKANFKIRHNVEDGSVQL ADHYQQNTPIGDGPVLLPDNHYLSTQSKLSKDPNEKRDHMVLLEF VTAAGITHGMDELYKSGGLPETGGHHHHHHV |
| mCherry -SRT-His6 | 22 | MVSKGEEDNMAIIKEFMRFKVHMEGSVNGHEFEIEGEGEGRPYEG TQTAKLKVTKGGPLPFAWDILSPQFMYGSKAYVKHPADIPDYLKL SFPEGFKWERVMNFEDGGVVTVTQDSSLQDGEFIYKVKLRGTNFP SDGPVMQKKTMGWEASSERMYPEDGALKGEIKQRLKLKDGGHYDA EVKTTYKAKKPVQLPGAYNVNIKLDITSHNEDYTIVEQYERAEGR HSTGGMDELYKSGGLPETGGHHHHHHV |
| sfGFP-Pep2-RXXGXX | 23 | MSMSKGEELFTGVVPILVELDGDVNGHKFSVRGEGEGDATNGKLT LKFICTTGKLPVPWPTLVTTLTYGVQCFARYPDHMKQHDFFKSAM PEGYVQERTISFKDDGTYKTRAEVKFEGDTLVNRIELKGIDFKED GNILGHKLEYNFNSHNVYITADKQKNGIKANFKIRHNVEDGSVQL ADHYQQNTPIGDGPVLLPDNHYLSTQSKLSKDPNEKRDHMVLLEF VTAAGITHGMDELYKSGGLPETGGGRRLEGKEEDEKGSRASDRFR GLR-[OH] |
| sfGFP-Pep2-OH | 24 | MSMSKGEELFTGVVPILVELDGDVNGHKFSVRGEGEGDATNGKLT LKFICTTGKLPVPWPTLVTTLTYGVQCFARYPDHMKQHDFFKSAM PEGYVQERTISFKDDGTYKTRAEVKFEGDTLVNRIELKGIDFKED GNILGHKLEYNFNSHNVYITADKQKNGIKANFKIRHNVEDGSVQL ADHYQQNTPIGDGPVLLPDNHYLSTQSKLSKDPNEKRDHMVLLEF VTAAGITHGMDELYKSGGLPETGGGRRLEGKEEDEKGSRASDDFR DLR-[OH] |
| sfGFP-Pep2-NH₂ | 25 | MSMSKGEELFTGVVPILVELDGDVNGHKFSVRGEGEGDATNGKLT LKFICTTGKLPVPWPTLVTTLTYGVQCFARYPDHMKQHDFFKSAM PEGYVQERTISFKDDGTYKTRAEVKFEGDTLVNRIELKGIDFKED GNILGHKLEYNFNSHNVY ITADKQKNGIKANFKIRHNVEDGSVQL ADHYQQNTPIGDGPVLLPDNHYLSTQSKLSKDPNEKRDHMVLLEF VTAAGITHGMDELYKSGGLPETGGGRRLEGKEEDEKGSRASDDFR DLR-[NH2] |
| sfGFP-Pep1-Ser-NH2 | 26 | MSMSKGEELFTGVVPILVELDGDVNGHKFSVRGEGEGDATNGKLTLKFICTTGKLP VPWPTLVTTLTYGVQCFARYPDHMKQHDFFKSAMPEGYVQERTISFKDDGTYKTR AEVKFEGDTLVNRIELKGIDFKEDGNILGHKLEYNFNSHNVYITADKQKNGIKANFKI RHNVEDGSVQLADHYQQNTPIGDGPVLLPDNHYLSTQSKLSKDPNEKRDHMVLL EFVTAAGITHGMDELYKSGGLPETGGGKDLEGKGGSAGSGSAGGSKYPYDVPDYA KS-[NH2] |
| mTagBFP 2-Pep2-RXXGXX | 27 | MVSKGEELIKENMHMKLYMEGTVDNHHFKCTSEGEGKPYEGTQTM RIKVVEGGPLPFAFDILATSFLYGSKTFINHTQGIPDFFKQSFPE GFTWERVTTYEDGGVLTATQDTSLQDGCLIYNVKIRGVNFTSNGP VMQKKTLGWEAFTETLYPADGGLEGRNDMALKLVGGSHLIANAKT TYRSKKPAKNLKMPGVYYVDYRLERIKEANNETYVEQHEVAVARY CDLPSKLGHKLNSGGLPETGGGRRLEGKEEDEKGSRASDRFRGLR -[OH] |
| mTagBFP 2-Pep2-OH | 28 | MVSKGEELIKENMHMKLYMEGTVDNHHFKCTSEGEGKPYEGTQTM RIKVVEGGPLPFAFDILATSFLYGSKTFINHTQGIPDFFKQSFPE GFTWERVTTYEDGGVLTATQDTSLQDGCLIYNVKIRGVNFTSNGP VMQKKTLGWEAFTETLYPADGGLEGRNDMALKLVGGSHLIANAKT TYRSKKPAKNLKMPGVYYVDYRLERIKEANNETYVEQHEVAVARY CDLPSKLGHKLNSGGLPETGGGRRLEGKEEDEKGSRASDDFRDLR -[OH] |
| mTagBFP 2-Pep2-NH₂ | 29 | MVSKGEELIKENMHMKLYMEGTVDNHHFKCTSEGEGKPYEGTQTM RIKVVEGGPLPFAFDILATSFLYGSKTFINHTQGIPDFFKQSFPE GFTWERVTTYEDGGVLTATQDTSLQDGCLIYNVKIRGVNFTSNGP VMQKKTLGWEAFTETLYPADGGLEGRNDMALKLVGGSHLIANAKT TYRSKKPAKNLKMPGVYYVDYRLERIKEANNETYVEQHEVAVARY CDLPSKLGHKLNSGGLPETGGGRRLEGKEEDEKGSRASDDFRDLR -[NH2] |
| mCherry-Pep2-RXXGXX | 30 | MVSKGEEDNMAIIKEFMRFKVHMEGSVNGHEFEIEGEGEGRPYEG TQTAKLKVTKGGPLPFAWDILSPQFMYGSKAYVKHPADIPDYLKL SFPEGFKWERVMNFEDGGVVTVTQDSSLQDGEFIYKVKLRGTNFP SDGPVMQKKTMGWEASSERMYPEDGALKGEIKQRLKLKDGGHYDA EVKTTYKAKKPVQLPGAYNVNIKLDITSHNEDYTIVEQYERAEGR HSTGGMDELYKSGGLPETGGGRRLEGKEEDEKGSRASDRFRGLR- [OH] |
| mCherry -Pep2-OH | 31 | MVSKGEEDNMAIIKEFMRFKVHMEGSVNGHEFEIEGEGEGR PYEGTQTAKLKVTKGGPLPFAWDILSPQFMYGSKAYVKHPA DIPDYLKLSFPEGFKWERVMNFEDGGVVTVTQDSSLQDGEF IYKVKLRGTNFPSDGPVMQKKTMGWEASSERMYPEDGALKG EIKQRLKLKDGGHYDAEVKTTYKAKKPVQLPGAYNVNIKLD ITSHNEDYTIVEQYERAEGRHSTGGMDELYKSGGLPETGGG RRLEGKEEDEKGSRASDDFRDLR-[OH] |
| mCherry -Pep2-NH₂ | 32 | MVSKGEEDNMAIIKEFMRFKVHMEGSVNGHEFEIEGEGEGR PYEGTQTAKLKVTKGGPLPFAWDILSPQFMYGSKAYVKHPA DIPDYLKLSFPEGFKWERVMNFEDGGVVTVTQDSSLQDGEF IYKVKLRGTNFPSDGPVMQKKTMGWEASSERMYPEDGALKG EIKQRLKLKDGGHYDAEVKTTYKAKKPVQLPGAYNVNIKLD ITSHNEDYTIVEQYERAEGRHSTGGMDELYKSGGLPETGGG RRLEGKEEDEKGSRASDDFRDLR-[NH2] |

Amino acids were loaded on chlorotrityl resin to access C-terminal carboxylic acids or Rink amide to access C-terminal amides. Automated peptide elongation was carried out on a Multisyntech Syro I parallel synthesizer according to the general peptide methods. Peptide was cleaved from the resin using TFA/DODT/H₂O (95:2.5:2.5, v/v) for 1 h. The resin was removed by filtration and the filtrate concentrated under reduced pressure. The solution was triturated with Et₂O and centrifuged to obtain crude peptide. The crude peptide was dissolved in H₂O/CH₃N (1:1, v/v) + 0.1% (v/v) TFA and purified using preparative HPLC.

### Recombinant production of sortase-tagged fluorescent proteins

Codon-optimized fluorescent protein cDNAs carrying tandem C-terminal Sortase-A and hexahistidine tags were synthesized (Integrated DNA Technologies) and cloned into the pET28 backbone by Gibson Assembly (NEBuilder HiFi DNA Assembly Master Mix, NEB). E coli BL21 (DE3) cells transformed with each expression vector were grown in terrific broth (LLG) to OD600 = 0.6 and recombinant proteins were expressed for 6h at 37°C in presence of 0.5 mM Isopropyl-β-D-thiogalactoside (IPTG). Proteins were extracted by sonication (Branson) in low-salt nickel buffer (150 mM NaCl, 20 mM Tris, 5% Glycerol, 25 mM imidazole, pH8) supplemented with Leupeptin (1 µg/ml), Pep-statin A (1 µg/ml) and PMSF (0.5 mM). Lysates were cleared by centrifugation (30 min, 40'000 g, 4°C) and fluorescent proteins were purified on a HisTrap FF column (Cytiva) by elution with 250 mM imidazole.

### Conjugation of synthetic peptide to fluorescent proteins using sortase

Peptide was dissolved in sortase reaction buffer (50 mM Tris, 150 mM NaCl, pH 7.4 at 4 °C). pH was adjusted to 7-8 using 2 M NaOH. Peptide (final concentration 1 mM) was mixed with mCherry/GFP (final concentration 75 µM). Sortase was added (final concentration 2 µM) and incubated at 4 °C for 18 h. Unreacted mCherry/GFP and cleaved sortag were removed by Ni-NTA purification. The flow-through was collected and immediately buffer exchanged to ion exchange buffer (25 mM Tris, pH 8.5) using a desalting column (Cytiva). The sample was further purified by anion exchange using a MonoQ column (Cytiva) with a gradient of 0-25% high salt buffer (25 mM Tris, 1 M NaCl, pH 8.5) in 25 column volumes. Fractions containing the product were pooled, buffer exchanged to sortase reaction buffer (supplemented with 0.5 mM TCEP) and concentrated.

### Cell culture and fluorescent reporter stability assays

HEK293T cells were cultured in DMEM containing Glutamax (Thermo Fisher Scientific) and K562 cells were cultured in RPMI 1640 containing Glutamax (Thermo Fisher Scientific) under standard conditions. Growth media were further supplemented with 10% (v/v) Fetal Bovine Serum (Thermo Fisher Scientific) and 100 U/ml Penicillin-Streptomycin (Thermo Fisher Scientific). Stably transduced cells were selected using 2 µg/ml Puromycin (Thermo Fisher Scientific), 500 µg/ml Gene-ticin (Thermo Fisher Scientific) or 10 µg/ml Blasticidin (Thermo Fisher Scientific) starting two days after transduction. Induction of doxycycline-dependent vectors was performed using 500 ng/ml doxycycline (Merck) every 48 hours. Cells were treated with epoxomicin (Merck), concanamycin A (Merck) or TAK243 (MedChem Express) as indicated.

For measuring stability of recombinant or semi-synthetic proteins, 1.5- 2.0 x 10^5 cells were nucleofected with 200 pmol of protein in 20 µl cuvettes using a 4D nucleofector device (Lonza) according to the manufacturer's recommendations. Following protein delivery, cells were left to recover at 37°C for 30-45 minutes before measuring initial mean fluorescence intensity values (t0) on a Attune NxT Flow Cytometer (Thermo Fisher Scientific). Subsequent measurements were performed at indicated time points. Baseline-fluorescence values of mock nucleofected cells receiving no protein were measured and subtracted for each time point and baseline-corrected fluorescence values were normalized to t0.

### Screen for modified amino acid degrons

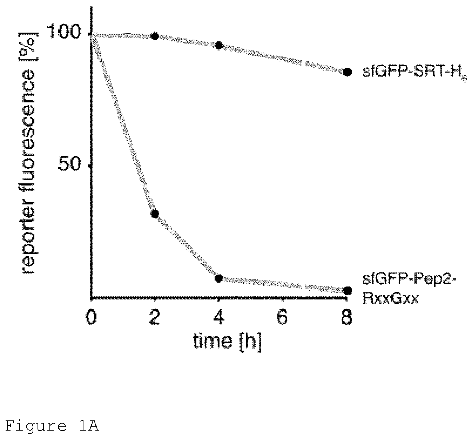

The effect of each modification on protein degradation was measured in the human erythroleukemia cell line K562, which allows for efficient and uniform protein delivery via nucleofection. Flow cytometry measurements showed that sortase-tagged sfGFP alone was highly stable (t1/2 > 16h, while introduction of a strong degron motif derived from the human ASCC3 C-terminus (-RXXGXX) led to rapid protein degradation. Figure 1A shows a protein stability assay in K562 cells nucleofected with the unconjugated sfGFP reporter (sfGFP-SRT-His6; SEQ ID No. 21) or sfGFP carrying a C-terminal degron motif (sfGFP-Pep2-RXXGXX; SEQ ID No. 23). Figure 1B shows the validation of terminal amidation as a degradation-inducing modification using an independent peptide context (Pep2) with (sfGFP-Pep2-NH2, SEQ ID No. 25) or without terminal amide (sfGFP-Pep2-OH, SEQ ID No. 24) in a protein degradation time-course as in (b).

sfGFP carrying a primary amide on its C-terminus (sfGFP-Pep1-Ser-NH2; SEQ ID No. 26) was strongly destabilized in the context of the original screening peptide (Figure 1B).

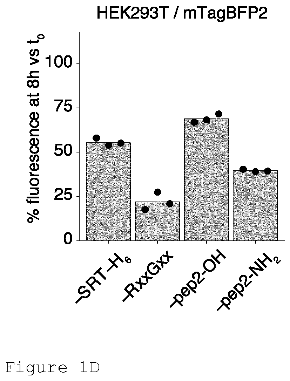

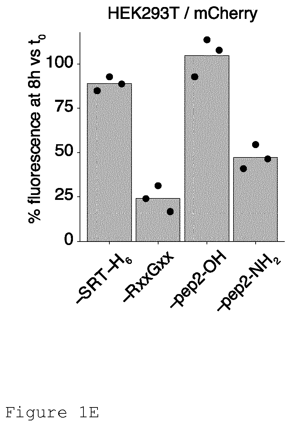

This effect could be fully blunted by inhibitors of total cellular ubiquitylation (TAK243) or the proteasome (epoxomicin), but not by inhibition of lysosomal acidification (concanamycin A) (Fig. 1C), suggesting that C-terminally amidated proteins are actively cleared from cells by the ubiquitin proteasome system. Lastly, conjugates of mTagBFP2 and mCherry carrying a primary C-terminal amide were also efficiently degraded when delivered to human embryonic kidney-derived HEK293T cells (Fig.1D and Fig 1E) establishing that C-terminal amidation induces protein degradation in different amino acid-, protein- and cell contexts.

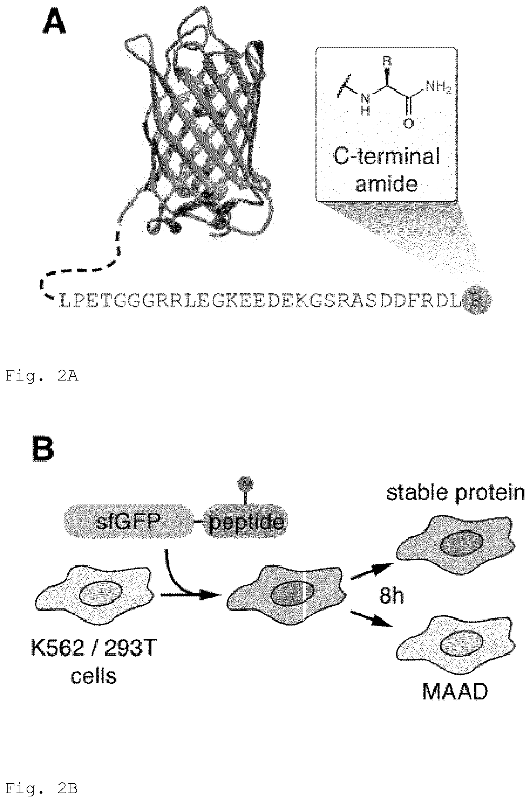

In line with what has been discussed above, a set of semi-synthetic fluorescent reporter proteins bearing candidate modifications were generated and introduced into cells to measure their turnover and ultimately to determine the effect of individual chemical modifications of proteins on their degradation in human cells. The concept is shown in Fig. 2A and 2B, in which Fig. 2A relates to a schematic of an sfGFP-based semi-synthetic reporter protein carrying a C-terminal amide, and Fig. 2B relates to a schematic of a fluorescent in-cell reporter assay to distinguish modified amino acid degrons (MAADs) from neutral modifications by electroporation of reporter proteins into human cell lines.

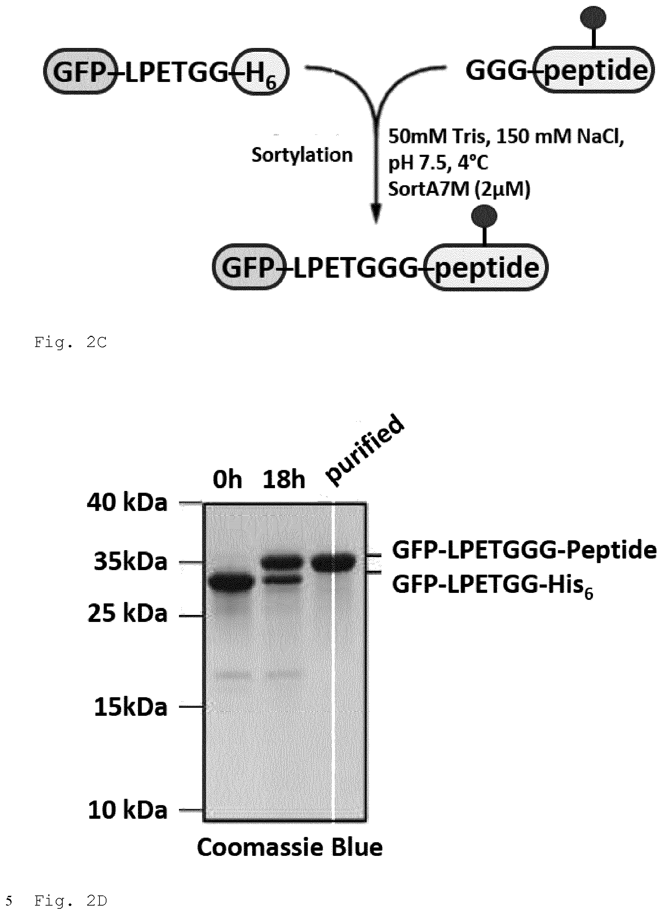

Using solid phase peptide synthesis, a set of peptides carrying defined modifications that represent different types of protein damage was generated: tyrosine modification through oxidation or misincorporation (L-3,4-dihydroxyphenylalanine, L-DOPA), carbonylation (N(6)-hexanoyllysine), advanced glycation end products (N(6)-carboxymethyllysine), carbamylation (homocitrulline) and primary amide-forming backbone cleavage (C-terminal amide). Sortase A-mediated conjugation of modified peptides onto the C-terminus of recombinant fluorescent proteins yielded a set of chemically defined modified reporters for their subsequent study in cells, as shown in Fig. 2C and 2D, of which Fig. 2C relates to a schematic showing modification of sfGFP with a C-terminal sortylation tag, and Fig. 2D relates to an exemplary SDS-PAGE analysis of sortase reaction showing sfGFP, crude reaction mixture and purified sfGFP-conjugate.

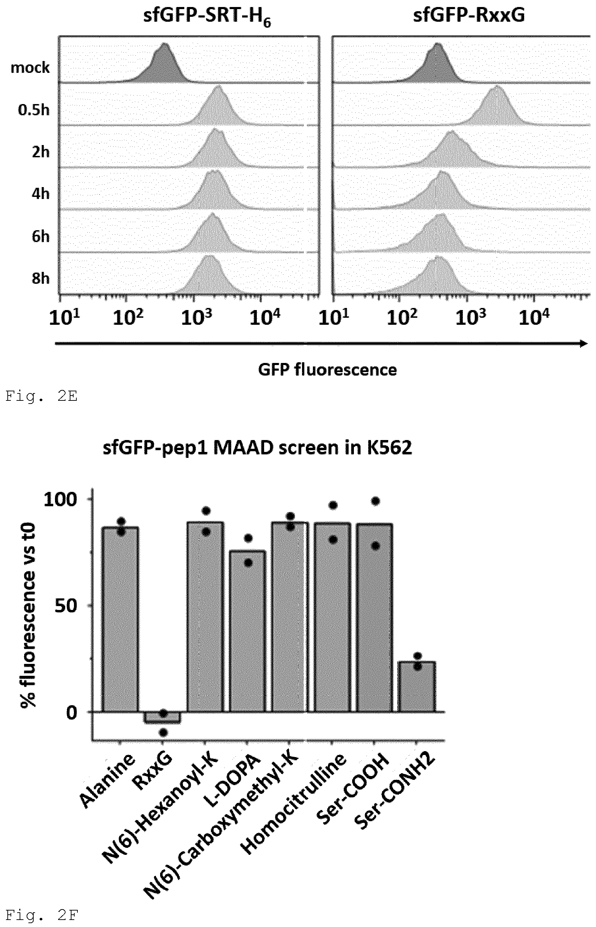

The effect of each modification on protein degradation in the human erythroleukemia cell line K562 was measured. A fluorescent in-cell reporter assay was performed, in which individual semi-synthetic proteins were introduced by electroporation into human cells and their clearance over time was followed by flow cytometry. In this setting, sortase-tagged superfolder GFP (sfGFP-SRT-H6) alone was highly stable (t1/2 > 16h), while introduction of a strong degron sequence derived from the human ASCC3 C-terminus (RxxG) induced rapid degradation (see Fig. 2E relating to an exemplary time-course experiment measuring sfGFP turnover in K562 cells, in which cells received sfGFP carrying a C-terminal sortase tag or its conjugated form carrying an RxxG degron motif). None of the internal modifications tested affected protein stability in this cell line and amino acid context, suggesting that they are not universally sufficient to induce protein degradation (see Fig. 2F).

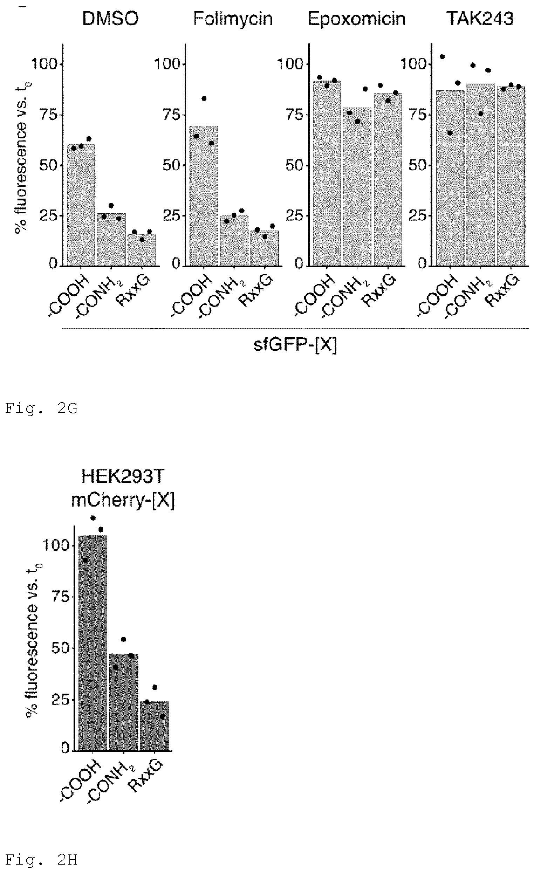

In contrast, sfGFP carrying a primary amide on its C-terminus was rapidly degraded in two independent sequence contexts, as shown in Fig. 2G, in which RxxG denotes an sfGFP variant containing a positive control degron motif, and which relates to experiments, in which K562 cells received mock-treatment (DMSO), lysosomal inhibitor Folimycin (100 nM), proteasome inhibitor epoxomicin (500 nM) or E1 ubiquitin ligase inhibitor TAK243 (1 µM) upon sfGFP delivery. Amidated sfGFP degradation was prevented by inhibitors of total cellular ubiquitylation (TAK243) or the proteasome (epoxomicin), but not by inhibition of lysosomal acidification (folimycin; see also the explanations above relating to Fig. 1C). This implies that C-terminal amide-bearing proteins (CTAPs) are actively cleared from cells by the ubiquitin proteasome system. CTAP forms of both mCherry and mTagBFP2, but not their unmodified counterparts, were also efficiently degraded when delivered to human embryonic kidney-derived HEK293T cells, as shown in Fig. 2H (see also the explanations above relating to Fig. 1E). The presence of a C-terminal amide is therefore sufficient to induce protein degradation in different peptide-, protein- and cell contexts.

In the following, the general materials and methods of the working examples are outlined with referring to the figures attached.

### Identification of the underlying machinery

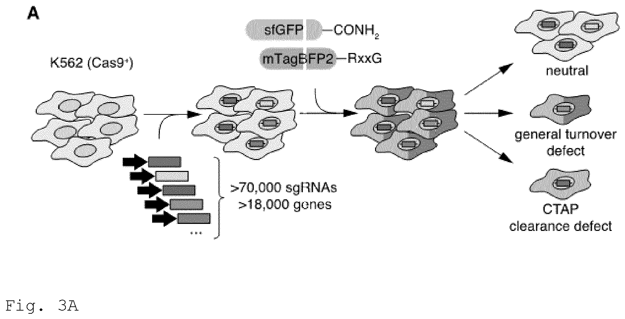

To identify the cellular machinery underlying the recognition and removal of C-terminally amidated proteins (CTAPs), a genome-wide CRISPR screen for genes responsible for specific degradation of C-terminally amidated sfGFP (sfGFP-CONH2) was devised, as discussed above. The concept is illustrated in Fig. 3A. Since knockout of central protein quality control and -turnover genes may impede cell survival, a clonal K562 cell line with a tightly controllable Cas9 allele (iCas9) was generated. Following transduction with a previously developed TKOv3 genome-wide sgRNA library, Cas9 expression was induced for 5 days to allow for efficient knockout while minimizing loss of cells with defects in essential pathways. Next, a C-terminally amidated GFP variant was delivered by electroporation together with mTagBFP2-RxxG, an internal control protein for general protein turnover carrying a sequence-based degron. Following the onset of protein degradation (14 h), cells that were proficient in general protein turnover but no longer cleared the CTAP (BFP⁻GFP⁺) were isolated, as well as a control population (BFP⁻GFP⁻).

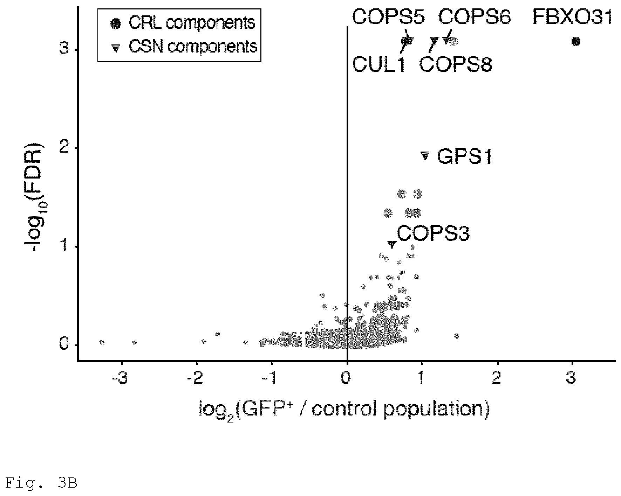

Bioinformatic analysis revealed a stark enrichment of few targeted genes in the CTAP clearance-deficient population. As shown in Fig. 3B, the most prominent hit was FBXO31, which is a substrate adaptor for the SCF (SKP1-CUL1-F-box protein) E3 ubiquitin ligase assembly.

### Validation of the role of FBXO31 in CTAP clearance

### - Knock-down

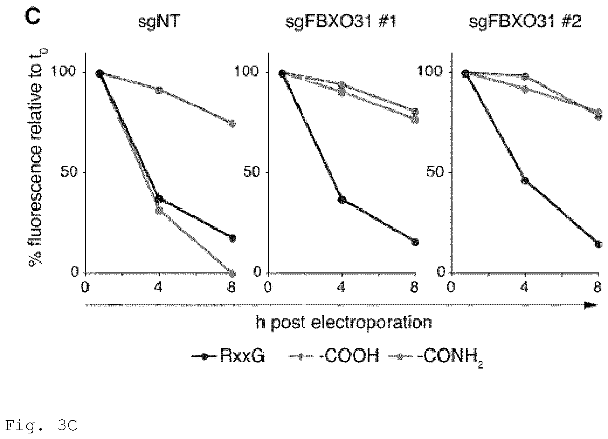

To test whether SCF/FBXO31 mediates CTAP clearance in an orthogonal assay, FBXO31 was knocked down using CRISPR inhibition (CRISPRi) and the degradation of an sfGFP conjugate with a different C-terminal sequence was measured. The results are given in Fig. 3C, which shows that FBXO31-targeting sgRNAs completely stabilized the amide-form of this reporter, while a reporter carrying the RxxG degron remained unaffected.

### - Fluorescence polarization

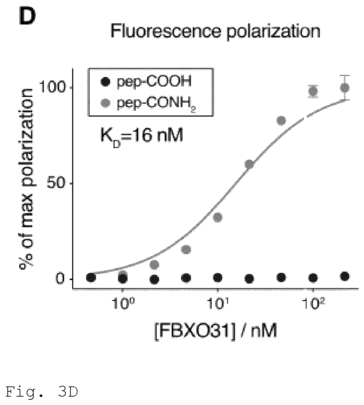

It was then tested whether SCF/FBXO31 directly binds and ubi-quitylates amidated clients, or whether it plays an indirect role in CTAP clearance. To this end, recombinant FBXO31 in complex with the binding partner SKP1 was purified and its affinity for various peptides was measured by fluorescence polarization (FP). In vitro, FBXO31 bound the peptide used for screening with high affinity (KD = 16 ± 2nM) while no binding could be detected for its carboxylic acid form, as shown in Fig. 3D.

### - Reconstitution of ligase assembly

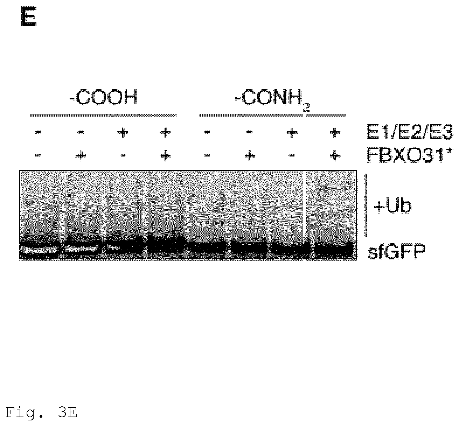

To test whether FBXO31 binding leads to productive ubiquitylation of substrates, the full SCF/FBXO31 E3 ligase assembly was reconstituted from recombinant components. Indeed, the SCF/FBXO31 complex ubiquitylated sfGFP-CONH2 in vitro, but had no detectable activity on sfGFP-COOH, as shown Fig. 3E. Together these results establish that SCF/FBXO31 is a reader of C-terminal protein amides and that this amide is required for SCF/FBXO31 to ubiquitylate targets.

### - Alteration of substrate specificity by cerebral-palsy associated mutation

Based on the finding of a dominant de novo D334N mutation in FBXO31 among patients with diplegic spastic cerebral palsy, this mutation was speculated to act by increased degradation of Cyclin D1 and eliminates the negative charge promoting CTAP recognition. It was therefore assessed whether the D334N mutation alters FBXO31 substrate recognition and CTAP40 clearance.

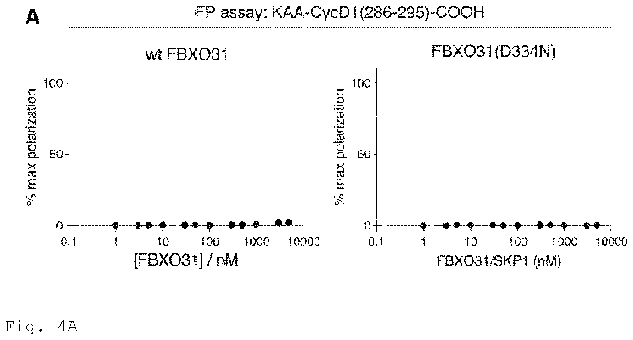

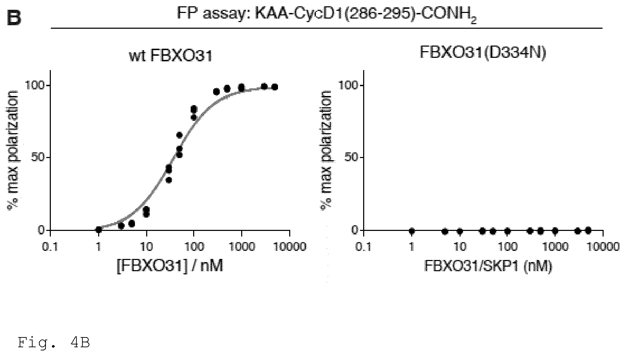

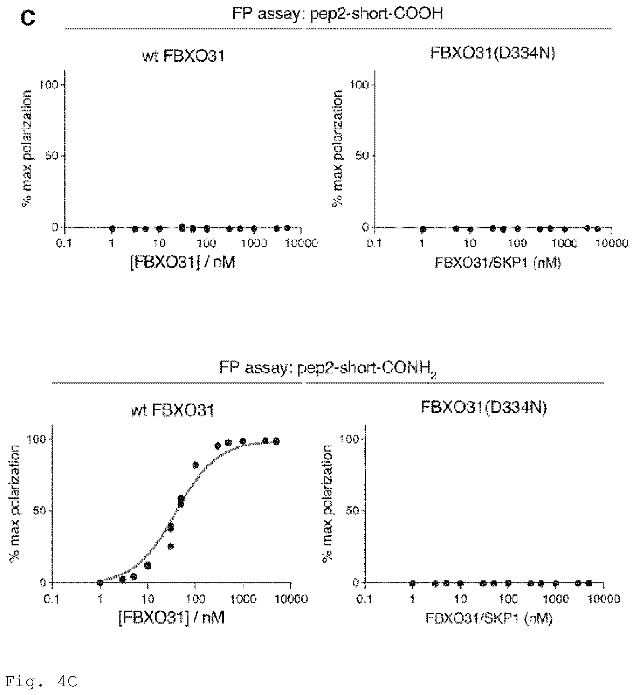

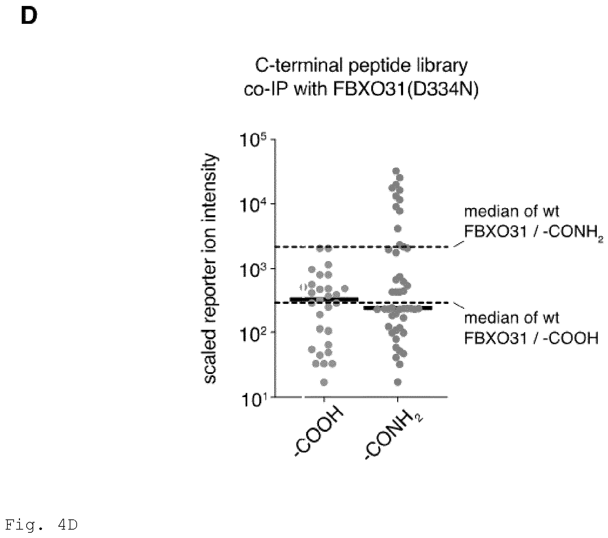

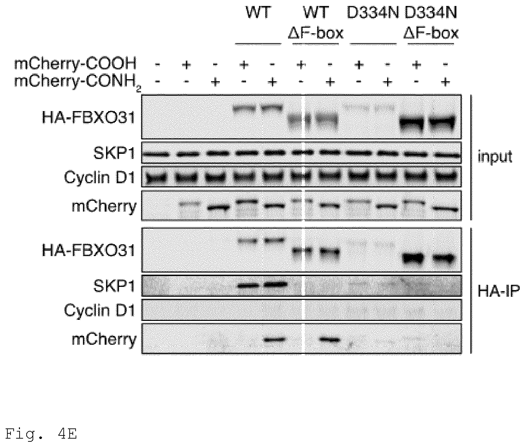

In vitro, neither wild type nor D334N mutant FBXO31 showed any affinity for the proposed C-terminal degron of Cyclin D1, as shown in Fig. 4A. However, the D334N mutation abolished binding to C-terminal amide peptides (Fig. 4B and C). This also held true in a pooled peptide interaction screen covering >1200 peptides, where the D334N mutation displayed globally reduced CTAP binding (Fig. 4D). Likewise, FBXO31(D334N, ΔF-box) expressed in FBXO31 knockout cells failed to immuno-precipitate both a model CTAP (mCherry-CONH2) and unmodified Cyclin D1, as shown in Fig. 4E.

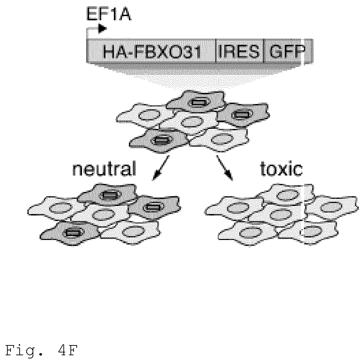

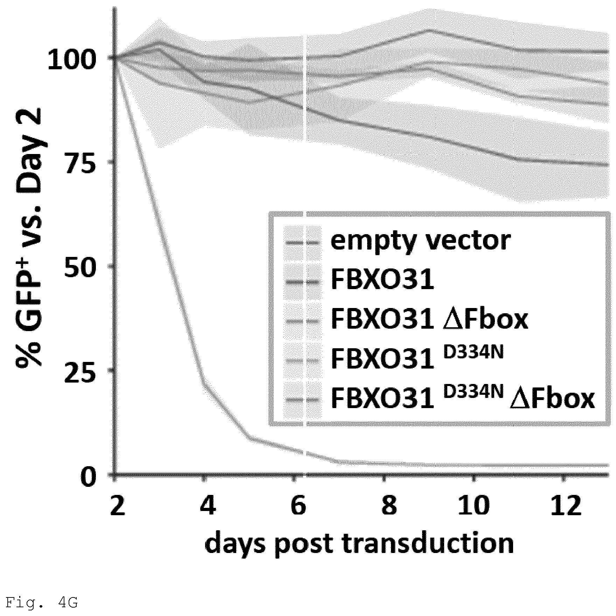

Based on the finding that full-length FBXO31(D334N) could not be stably expressed over extended culture periods, even in cells expressing wildtype FBXO31, a competitive growth assay was performed to quantitatively test whether FBXO31(D334N) compromises cell survival, the results of which being shown in Fig. 4F. While wildtype FBXO31 cDNA expression was well tolerated in FBXO31 knockout HEK293T cells, the D334N mutant was rapidly depleted from co-culture, as shown in Fig. 4G. Deletion of the F-box motif required for SCF complex assembly fully abolished this effect, suggesting that FBXO31(D334N) exerts a toxic ubiquitin ligase activity.

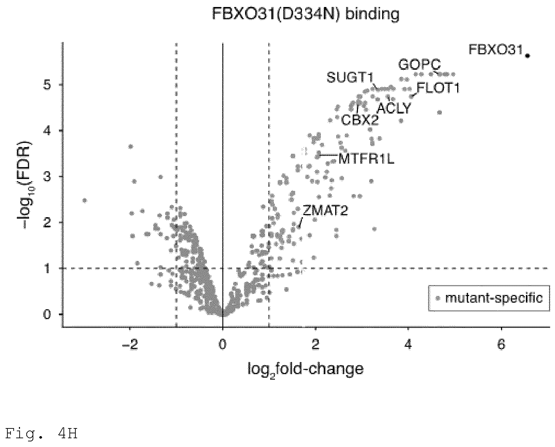

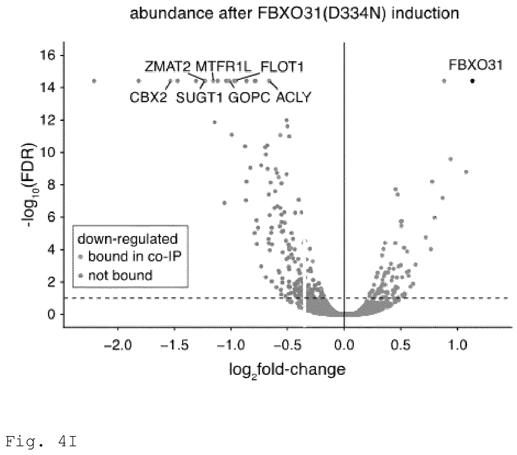

Co-IP MS of FBXO31(ΔF-box) was performed using both the wildtype and D334N mutant to determine how the mutation alters substrate recognition. FBXO31(D334N, ΔF-box) formed detectable interactions with 220 proteins, 195 of which were not detected for the wildtype (Fig. 4H). It was tested whether these putative neo-substrates are down-regulated in response to acute FBXO31(D334N) expression using the ligand-inducible shield-degron system. Tandem mass tag (TMT) expression pro-teomics identified a marked reduction in the abundance of several candidates within 12h of inducing DD-FBXO31(D334N), but not the wildtype (Fig. 4I). Among these neo-substrates were core-essential proteins (ACLY, SUGT1 and PRDX2), which could account for the observed proliferation defect. Based on these results it can be concluded that D334 is required for CTAP binding, and the cerebral-palsy associated mutation is dominant because it redirects ubiquitin ligase activity away from C-terminal amide substrates and towards multiple essential cellular proteins.

### Characterization of binding of FBXO31 to CTAP

To determine the substrate scope of FBXO31, in vitro binding studies as discussed in the following were carried out:
In this regard, it was first tested whether FBXO31 specifically binds C-terminal amides rather than side chain amides in asparagine or glutamine. FP assays using recombinant FBXO31/SKP1 and fluorescently labeled peptides showed no affinity for peptides with unmodified N or Q at the C-terminus.

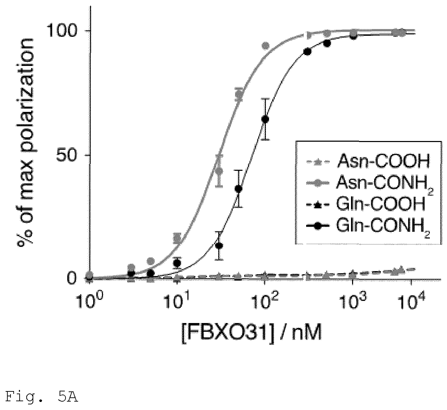

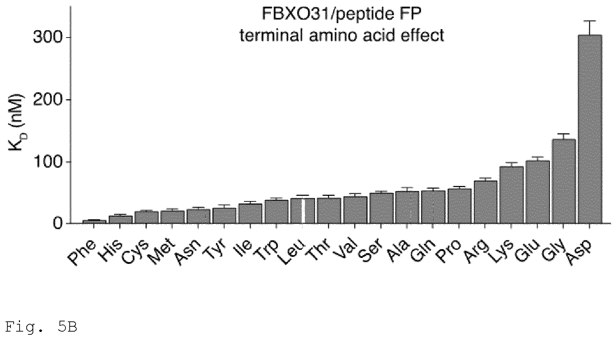

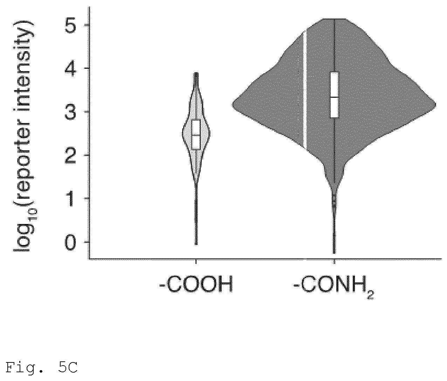

However, the same peptide sequences were bound with high affinity when carrying a C-terminal primary amide (X-N-CONH2: KD = 24 ± 3 nM, X-Q-CONH2: KD = 55 ± 4 nM), as shown in Fig. 5A. Extending this assay to peptides bearing the primary amide derivatives of each of the 20 natural amino acids revealed that FBXO31 can bind virtually any C-terminal amide with nanomolar affinity, as shown in Fig. 5B. The weakest binders were peptides bearing glycine and acidic residues, with X-D-CONH2 exhibiting a KD of 304 ± 22 nM. Hydrophobic residues were most strongly bound, with X-F-CONH2 as the best substrate (KD ≈ 6 nM), followed by other and then uncharged and charged hydrophilic side chains. These findings demonstrate that FBXO31 binds diverse C-terminal amides with high affinity and selectivity over unmodified C-termini and side chain amides. Individual testing of amidated C-termini was extended by devising a massively parallel protein-peptide interaction screen to identify rules of the broader substrate preference by FBXO31. Using isokinetic mixtures of 19 natural amino acids (all except cysteine) in the first three coupling steps, peptide libraries containing >2'000 distinct C-termini detectable by mass spectrometry (MS) were synthesized. To quantify FBXO31/SKP1 binding to these sequences, in vitro co-immuno-precipitation of the library was performed and the abundance of each peptide alongside the input pool was quantified using isobaric labeling and MS. Overall, 841 distinct C-terminal amides co-purified with FBXO31, compared to just 73 unmodified C-termini. In addition, the C-terminal amides also showed 7.6-fold greater enrichment compared to unmodified peptides (Fig. 5C). Compared to the input library, FBXO31-bound peptides were enriched for hydrophobic side chains, while acidic residues were disfavored, especially in the terminal position. Despite these preferences, each tested amino acid could be detected in any of the three ultimate positions among bound peptides. The overall conclusion was drawn that FBXO31 is specific for peptide amidation and disfavors negatively charged termini. Unlike conventional sequence-based C-degrons FBXO31 is rather agnostic to specific sequence motifs, potentially enabling it to broadly surveil C-terminal amides across the diverse proteome.

The specific methods for generating the FBXO31 knockout cells and for performing the CRISPR screen and next generation sequencing are described in the following in more detail:

### Gene editing

FBXO31 knockout cells were generated by electroporation of cells with Cas9/sgRNA ribonucleoprotein particles as described previously. In brief, in vitro transcription templates were generated by PCR using Q5 polymerase (New England Biolabs) and primers listed in supplementary table S1 and used for in vitro transcription by T7 RNA polymerase (NEB).

| Oligo_ID | SEQ ID NO. : | Sequence | note |
|---|---|---|---|
| genomic DNA amplification | | | |
| TKOv3_{_}PCR1_ pLCK02_fwd | 33 | GAGGGCCTATTTCCCATGATTC | PCR1 of TKOv3 sgRNA library from genomic DNA |
| TKOv3 _PCR1_{_} pLCKO2_rev | 34 | CAAACCCAGGGCTGCCTTGGAA | PCR1 of TKOv3 sgRNA library from genomic DNA |

| deep sequencing library amplification | | | |
|---|---|---|---|
| TS_ampli-con _NGS_fwd | 35 | AATGATACGGCGACCACCGAGATCTACACG AGAGGTTACACTCTTTCCCTACACGACGCT CTTCCGATCT | generation of NGS libraries from genomic DNA amplicons (FBXO31.KO 3-F1/R1) |
| TS_ampli-con_NGS_rev | 36 | CAAGCAGAAGACGGCATACGAGATGGATCT GAGTGACTGGAGTTCAGACGTGTGCTCTTC CGATC | generation of NGS libraries from genomic DNA amplicons (FBXO31.KO 3-F1/R1) |
| TKOv3_PCR2_ i5(502) _fwd | 37 | AATGATACGGCGACCACCGAGATCTACACG ATATCGAACACTCTTTCCCTACACGACGCT CTTCCGATCTTTGTGGAAAGGACGAGGTAC CG | PCR2 of TKOv3 sgRNA library for deep sequencing |
| TKOv3 _PCR2_ i5(503) fwd | 38 | AATGATACGGCGACCACCGAGATCTACACC GCAGACGACACTCTTTCCCTACACGACGCT CTTCCGATCTTTGTGGAAAGGACGAGGTAC CG | PCR2 of TKOv3 sgRNA library for deep sequencing |
| TKOv3_PCR2_ i7(701) rev | 39 | CAAGCAGAAGACGGCATACGAGATCCGCGG TTGTGACTGGAGTTCAGACGTGTGCTCTTC CGATCTACTTGCTATTTCTAGCTCTAAAAC | PCR2 of TKOv3 sgRNA library for deep sequencing |
| TKOv3_PCR2_ i7(704)_rev | 40 | CAAGCAGAAGACGGCATACGAGATAAGTCC AAGTGACTGGAGTTCAGACGTGTGCTCTTC CGATCTACTTGCTATTTCTAGCTCTAAAAC | PCR2 of TKOv3 sgRNA library for deep sequencing |

Resulting RNA was purified using a spin-column kit (RNeasy mini kit, QIAGEN) and 120 pmol of sgRNA were complexed with 100 pmol of recombinant SpCas9 protein at RT for 20 minutes. Cas9 protein was obtained from the QB3 Macro Lab at UC Berkeley. Assembled sgRNA/Cas9 complexes were delivered to cells using a 4D nucleofector kit (Lonza) according to the manufacturer's instructions. Clonal cell lines were isolated by single-cell sorting using an SH-800 cell sorter (Sony) and characterized by genomic DNA extraction (Lucigen QuickExtract), PCR amplification of edited loci using Q5 polymerase (NEB) with genotyping and NGS primers listed above. Pooled next generation sequencing of edited loci was performed by the Genome Engineering and Measurement Lab of the Functional Genomics Center Zürich on a MiSeq sequencer (Illumina) with 150 bp paired-end reads. Deep sequencing reads were analyzed using CRISPResso2 (Clement, K. et al. (2019) 'CRISPResso2 provides accurate and rapid genome editing sequence analysis', Nature Biotechnology, 37(3), pp. 224-226).

### Viral transduction and knockdown

Lentiviral vectors were packaged in HEK293T cells using standard methods (Stewart, S. A. et al. (2003) 'Lentivirus-delivered stable gene silencing by RNAi in primary cells', Rna, 9(4), pp. 493-501). In brief, cells were incubated with plasmid DNA (transfer plasmid, pCMV-dR8.2 dvpr and pCMV-VSV-G at a weight-ratio of 4:2:1) and Polyethyleneimine (Mw ~25000 u) at a 1:3 weight ratio (total DNA to PEI). Viral supernatant was harvested at 48-72 hours post nucleofection by ultrafiltration and supplemented with 4 µg/ml polybrene.

Puromycin was used to select for cells stably expressing sgRNAs. To validate knockdown efficiency, stably transduced sgRNA-expressing cells were harvested for RNA extraction (RNeasy Mini Kit, QIAGEN), reverse transcription (iScript Reverse Transcription Supermix, BioRad) and quantitative PCR (SsoFast EvaGreen Supermix, BioRad) analyzed on a QuantStudio 6 thermocycler (Thermo Fisher Scientific) using the ΔΔCT method.

### Generation of CRISPR- and CRISPRi-competent cell lines

K562 cells competent for inducible gene knockout (iCas9) and genome-wide CRISPR screening were generated by transduction with vectors SRPB (pHR-SFFV-rtTA3-PGK-Bsr) and 3GCasT (pHR-TRE3G-hSpCas9-NLS-FLAG-2A-Thyl.1). Cas9-P2A-Thy1.1 expression was induced using doxycycline for 2 days and cells staining positive for Thy1.1 were isolated by single-cell sorting (Sony SH-800). Clonal lines were screened for cells that show no evidence of CD55 knockout following viral delivery of sgCD55.1 and efficient knockout following addition of doxycycline for 9 days, each by antibody staining and flow cytometry.

### CRISPR screen and next generation sequencing

iCas9 cells were transduced with the pooled lentiviral sgRNA library TKOv3 (Hart, T. et al. (2017) 'Evaluation and Design of Genome-Wide CRISPR/SpCas9 Knockout Screens.', G3 (Bethesda, Md.), 7(8), pp. 2719-2727) at a multiplicity of infection (MOI) of about 0.3 as measured by serial dilution, puromycin selection and viability assay (CellTiter-Glo 2.0, Promega). Two pools of 1.2 · 10^8 cells each were transduced, yielding ca 500-fold library coverage which was maintained throughout all cell culture steps. Cas9-expression was induced by addition of doxycycline for 5 days prior to delivery of reporter proteins to allow for efficient knockout while minimizing drop-out of essential genes. To isolate CTAP-clearance deficient cells ≥ 4 large-scale nucleofections were performed by combining 5 · 10^7 cells, 2000 p mol C-terminally amidated sfGFP (sfGFP-Pep2-NH2 SEQ ID No. 25) and 2000 p mol unstable control protein mTAGBFP-Pep2-RXXGXX; (SEQ ID No. 27) in a 100 µl nucleofection reaction (4D nucleofector kit SE plus supplement SF1, Lonza). 14 hours following nucleofection, cells were transferred on ice and sorted into a CTAP deficient population (sfGFP high concentration, mTagBFP2 low concentration) and an unaffected control population (sfGFP low concentration, mTagBFP low concentration). Genomic DNA was extracted from snap-frozen sorted cells using the Gentra

Pure kit (QIAGEN). sgRNA cassettes were isolated by two rounds of PCR using a previously published strategy with NEBNext Ultra II Q5 Master Mix (New England Biolabs) and primer pairs listed above.

Protospacers were quantified by deep sequencing using 21 initial dark cycles on a NovaSeq device (Illumina) by the Genome Engineering and Measurement Lab of the Functional Genomics Center Zürich (GEML, FGCZ). sgRNA counts were retrieved using mageck count (MAGeCK v0.5.9.3) with default parameters (Li, W. et al. (2014) 'MAGeCK enables robust identification of essential genes from genome-scale CRISPR/Cas9 knockout screens.', Genome biology, 15(12), p. 554). Enrichment of sgRNAs targeting the same gene in CTAP-deficient cells versus the control population was estimated using mageck test using a paired design for screening duplicates with option remove-zero both and otherwise default parameters.

## Claims

1. Method for identifying a modified amino acid degron (MAAD), the method including the following subsequent steps of
a. preparing an organic molecule of the general formula (I)
X-T-Z (I)
wherein
T is an organic moiety comprising at least one canonical or non-canonical amino acid,
X is a peptide comprising two or more canonical amino acids said peptide being covalently linked to T by an amide bond;
Z is a functional end group comprising a heteroatom selected from the group consisting of oxygen, nitrogen and sulfur;
with the proviso that if Z is OH, T does not consist of canonical amino acids;
b. preparing from the organic molecule of the general formula (I) a protein conjugate of the general formula (II)
P-(L)ₚ-T-Z (II)
wherein
P is a reporter protein,
T and Z have the same definition as in step a,
L is a peptide comprising two or more natural amino acids and p is 0 or 1;
c. incorporating the protein conjugate obtained in step b) in a cell line;
d. measuring the turnover of the protein conjugate and comparing it with the turnover of a control protein to identify if the protein conjugate is tagged with a MAAD inducing intracellular protein degradation,
e. generating a pool of mutant cells, each defective in a different gene, and
f. incorporating the MAAD-tagged protein conjugate identified in step d) into the pool of mutant cells generated in step e) to isolate mutant cells that fail to selectively degrade the protein conjugate, thus identifying factors mediating the selective degradation of the MAAD-tagged protein conjugate.

2. Method according to claim 1, wherein T is an organic moiety of the general formula (III)
-S-(Y)ₘ- (III)
wherein S is an organic moiety of the general formula (IV) 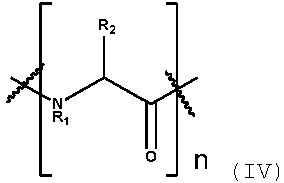 wherein
R₁ is hydrogen, a linear or branched, saturated or unsaturated C₁ to C₁₀ alkyl residue, or forms together with R₂ a ring system
R₂ is selected from the group consisting of hydrogen, substituted or unsubstituted alkyl, substituted or unsubstituted alkenyl, substituted or unsubstituted alkynyl, substituted or unsubstituted cycloalkyl, substituted or unsubstituted heterocyc-loalkyl, substituted or unsubstituted aryl, substituted or unsubstituted heteroaryl, substituted or unsubstituted aralkyl, substituted or unsubstituted aralkenyl, substituted or unsubstituted heteroaralkyl, and substituted or unsubstituted heteroaralkenyl,
n is 1 to 5,
Y is a canonical amino acid or a peptide comprising two or more canonical amino acids and m is 0 or 1.

3. Method according to claim 2, wherein n is 1 or 2, preferably 1.

4. Method according to claim 2 or 3, wherein R₂ is selected from the group consisting of substituted alkyl, substituted or unsubstituted alkenyl, substituted aryl, substituted or unsubstituted heteroaryl, substituted or unsubstituted aralkyl, substituted or unsubstituted aralkenyl, substituted or unsubstituted heteroaralkyl, and substituted or unsubstituted heteroaralkenyl, preferably substituted alkyl and substituted aralkyl.

5. Method according to any of claims 2 to 4, wherein S is a modified amino acid.

6. Method according to any of claims 2 to 5, wherein S comprises a non-canonical D-amino acid.

7. Method according to any of claims 2 to 6, wherein S is an enzymatically modified amino acid.

8. Method according to any of claims 2 to 6, wherein S is a chemically modified amino acid.

9. Method according to any of the preceding claims, wherein Z is selected from the group consisting of NH₂, SH₂, OMe and OEt, preferably NH₂ and OMe, most preferably NH₂.

10. Method according to claim 9, wherein S is a canonical amino acid and m is 0.

11. Method according to any of claims 2 to 10, wherein S is selected from the group consisting of
| | |
|---|---|
| 1 | 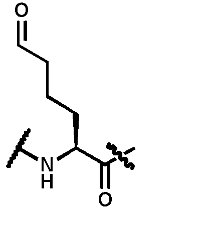 |
| 2 | 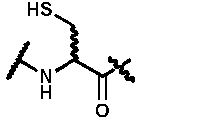 |
| 3 | 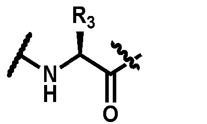 wherein R₃ is the residue of a canonical amino acid |
| 4 | 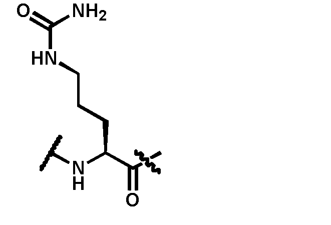 |
| 5 | 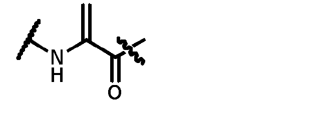 |
| 6 | 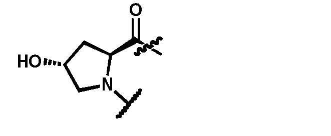 |
| 7 | 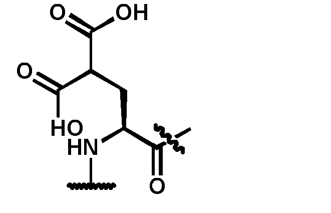 |
| 8 | 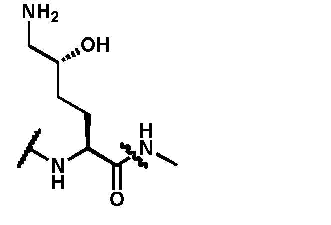 |
| 9 | 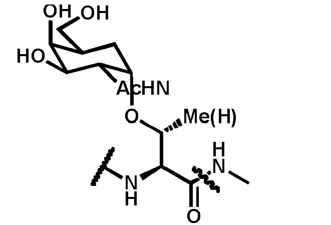 |
| 10 | 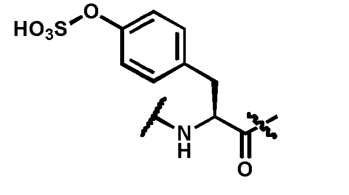 |
| 11 | 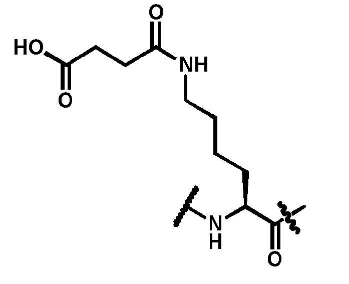 |
| 12 | 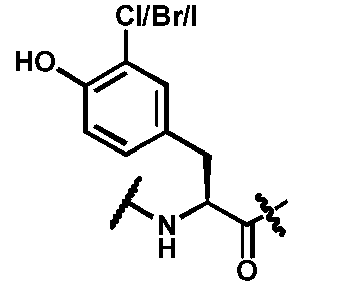 |
| 13 | 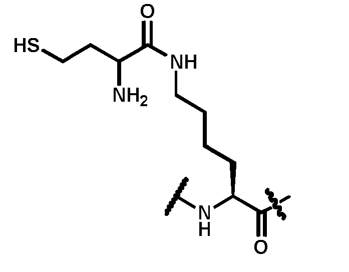 |
| 14 | 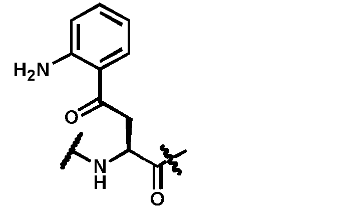 |
| 15 | 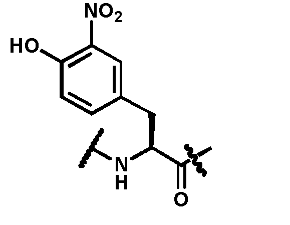 |
| 16 | 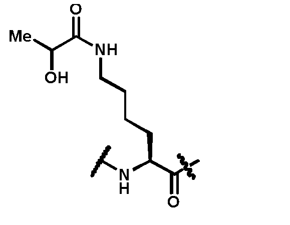 |
| 17 | 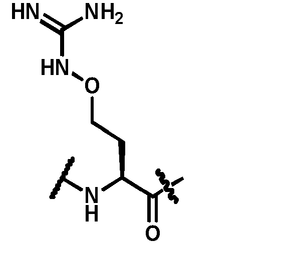 |
| 18 | 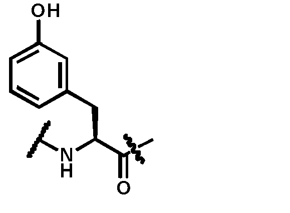 |
| 19 | 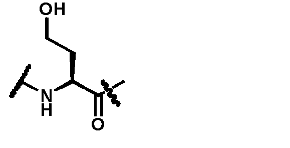 |
| 20 | 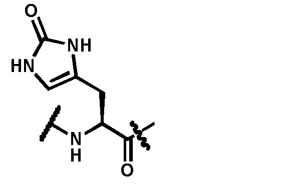 |
| 21 | 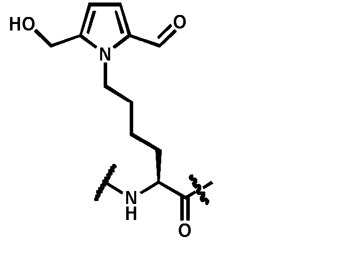 |
| 22 | 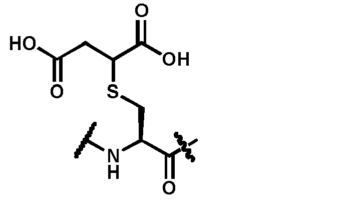 |
| 23 | 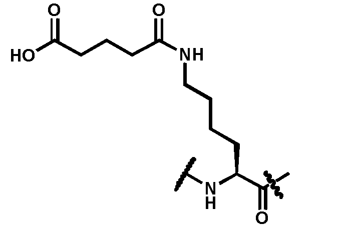 |

12. Method according to any of the preceding claims, wherein the preparation of the protein conjugate of step b) is performed by chemoenzymatically conjugating the compound of step a) to the reporter protein, in particular using sortase.

13. Method according to any of the preceding claim, wherein generating the pool of mutant cells according to step e) is performed using a CRISPR-based inducible knockout system, in particular using a doxycycline-dependent knockout system.

## Patentansprüche

1. Verfahren zur Identifizierung eines modifizierten Aminosäure-Degrons (MAAD), wobei das Verfahren die folgenden aufeinanderfolgenden Schritte umfasst:
a. Herstellung eines organischen Moleküls der allgemeinen Formel (I)
X-T-Z (I)
wobei
T eine organische Einheit ist, die mindestens eine kanonische oder nicht-kanonische Aminosäure umfasst,
X ein Peptid ist, das zwei oder mehr kanonische Aminosäuren umfasst, wobei das Peptid über eine Amidbindung kovalent an T gebunden ist;
Z eine funktionelle Endgruppe ist, die ein Heteroatom umfasst, das aus der Gruppe ausgewählt ist, die aus Sauerstoff, Stickstoff und Schwefel besteht;
mit der Massgabe, dass, wenn Z OH ist, T nicht aus kanonischen Aminosäuren besteht;
b. Herstellung eines Proteinkonjugats der allgemeinen Formel (II) aus dem organischen Molekül der allgemeinen Formel (I)
P-(L)ₚ -T-Z (II)
wobei
P ein Reporterprotein ist,
T und Z die gleiche Bedeutung wie in Schritt a haben,
L ein Peptid ist, das zwei oder mehr natürliche Aminosäuren umfasst, und p 0 oder 1 ist;
c. Einbringen des in Schritt b) erhaltenen Proteinkonjugats in eine Zelllinie;
d. Messen des Umsatzes des Proteinkonjugats und Vergleichen desselben mit dem Umsatz eines Kontrollproteins, um festzustellen, ob das Proteinkonjugat mit einem MAAD markiert ist, das einen intrazellulären Proteinabbau induziert,
e. Erzeugen eines Pools von Mutantenzellen, von denen jede einen Defekt in einem anderen Gen aufweist, und
f. Einbringen des in Schritt d) identifizierten MAAD-markierten Proteinkonjugats in den in Schritt e) erzeugten Pool von Mutantenzellen, um Mutantenzellen zu isolieren, die das Proteinkonjugat nicht selektiv abbauen, wodurch Faktoren identifiziert werden, die den selektiven Abbau des MAAD-markierten Proteinkonjugats vermitteln.

2. Verfahren nach Anspruch 1, wobei T eine organische Einheit der allgemeinen Formel (III) ist
-S-(Y)ₘ- (III)
wobei S eine organische Einheit der allgemeinen Formel (IV) ist 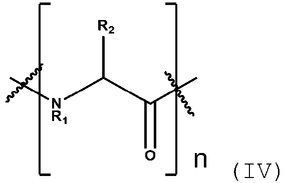 wobei
R₁ Wasserstoff, einen linearen oder verzweigten, gesättigten oder ungesättigten C₁- bis C₁₀- Alkylrest darstellt oder zusammen mit R₂ ein Ringsystem bildet,
R₂ ausgewählt ist aus der Gruppe bestehend aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Heterocycloalkyl, substituiertem oder unsubstituiertem Aryl, substituiertem oder unsubstituiertem Heteroaryl, substituiertem oder unsubstituiertem Aralkyl, substituiertem oder unsubstituiertem Aralkenyl, substituiertem oder unsubstituiertem Heteroaralkyl und substituiertem oder unsubstituiertem Heteroaralkenyl,
n 1 bis 5 ist,
Y eine kanonische Aminosäure oder ein Peptid ist, das zwei oder mehr kanonische Aminosäuren umfasst, und m 0 oder 1 ist.

3. Verfahren nach Anspruch 2, wobei n 1 oder 2 ist, vorzugsweise 1.

4. Verfahren nach Anspruch 2 oder 3, wobei R₂ ausgewählt ist aus der Gruppe bestehend aus substituiertem Alkyl, substituiertem oder unsubstituiertem Alkenyl, substituiertem Aryl, substituiertem oder unsubstituiertem Heteroaryl, substituiertem oder unsubstituiertem Aralkyl, substituiertem oder unsubstituiertem Aralkenyl, substituiertem oder unsubstituiertem Heteroaralkyl und substituiertem oder unsubstituiertem Heteroaralkenyl, vorzugsweise substituiertem Alkyl und substituiertem Aralkyl.

5. Verfahren nach einem der Ansprüche 2 bis 4, wobei S eine modifizierte Aminosäure ist.

6. Verfahren nach einem der Ansprüche 2 bis 5, wobei S eine nicht-kanonische D-Aminosäure umfasst.

7. Verfahren nach einem der Ansprüche 2 bis 6, wobei S eine enzymatisch modifizierte Aminosäure ist.

8. Verfahren nach einem der Ansprüche 2 bis 6, wobei S eine chemisch modifizierte Aminosäure ist.

9. Verfahren nach einem der vorstehenden Ansprüche, wobei Z ausgewählt ist aus der Gruppe bestehend aus NH₂, SH₂, OMe und OEt, vorzugsweise aus NH₂ und OMe, und am bevorzugtesten NH₂ ist.

10. Verfahren nach Anspruch 9, wobei S eine kanonische Aminosäure ist und m 0 ist.

11. Verfahren nach einem der Ansprüche 2 bis 10, wobei S ausgewählt ist aus der Gruppe bestehend aus
| | |
|---|---|
| 1 | 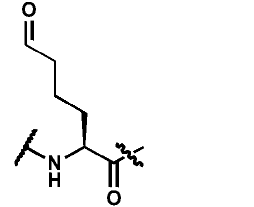 |
| 2 | 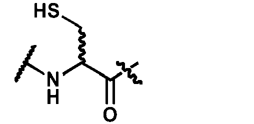 |
| 3 | 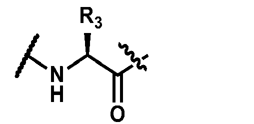 wobei R₃ der Rest einer kanonischen Aminosäure ist |
| 4 | 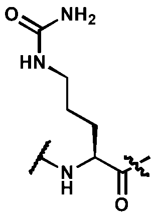 |
| 5 | 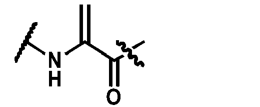 |
| 6 | 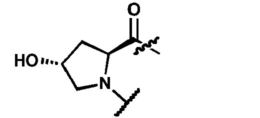 |
| 7 | 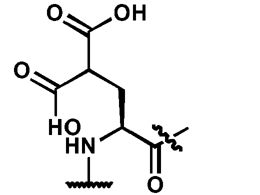 |
| 8 | 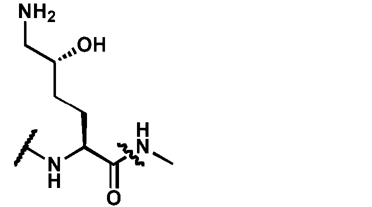 |
| 9 | 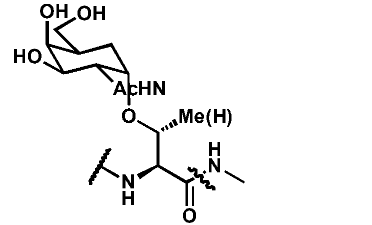 |
| 10 | 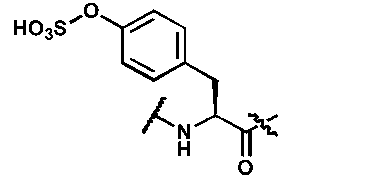 |
| 11 | 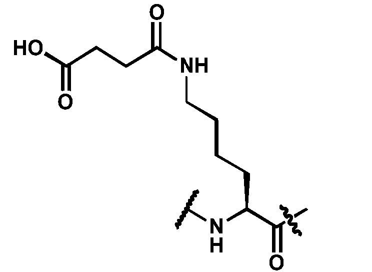 |
| 12 | 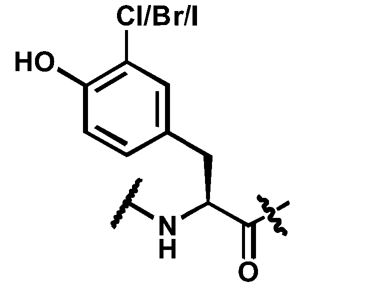 |
| 13 | 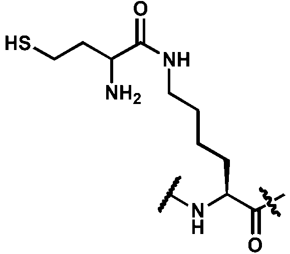 |
| 14 | 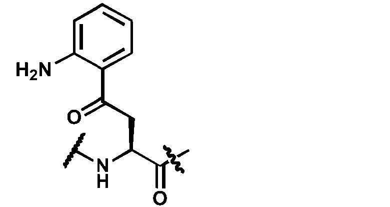 |
| 15 | 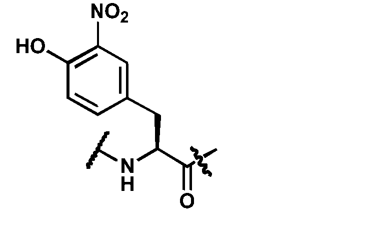 |
| 16 | 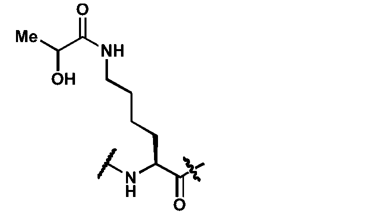 |
| 17 | 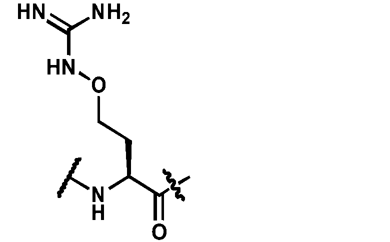 |
| 18 | 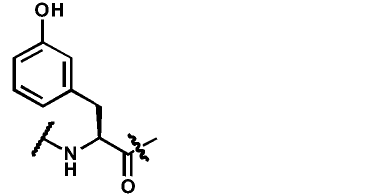 |
| 19 | 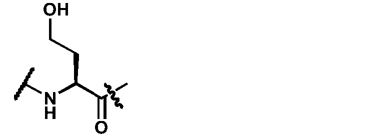 |
| 20 | 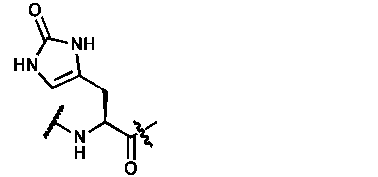 |
| 21 | 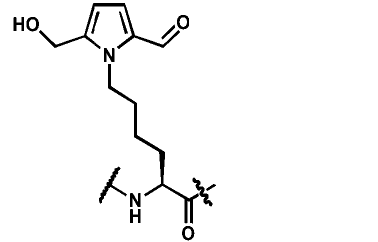 |
| 22 | 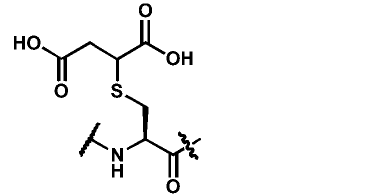 |
| 23 | 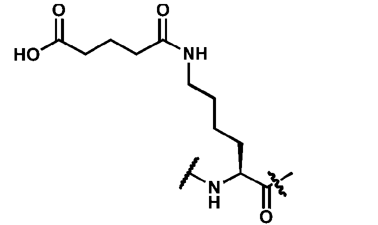 |

12. Verfahren nach einem der vorstehenden Ansprüche, wobei die Herstellung des Proteinkonjugats aus Schritt b) durch chemoenzymatische Konjugation der Verbindung aus Schritt a) an das Reporterprotein erfolgt, insbesondere unter Verwendung von Sortase.

13. Verfahren nach einem der vorstehenden Ansprüche, wobei die Erzeugung des Pools von Mutantenzellen gemäss Schritt e) unter Verwendung eines CRISPR-basierten induzierbaren Knockout-Systems durchgeführt wird, insbesondere unter Verwendung eines Doxycyclin-abhängigen Knockout-Systems.

## Revendications

1. Procédé d'identification d'un dégron d'acide aminé modifié (MAAD), ledit procédé comprenant les étapes successives suivantes consistant à
a. préparer une molécule organique de formule générale (I)
X-T-Z (I)
dans laquelle
T est un groupement organique comprenant au moins un acide aminé canonique ou non-canonique,
X est un peptide comprenant deux ou plusieurs acides aminés canoniques, ledit peptide étant lié de manière covalente à T par une liaison amide;
Z est un groupe terminal fonctionnel comprenant un hétéroatome choisi parmi le groupe constitué de l'oxygène, de l'azote et du soufre;
à condition que, si Z est OH, T ne soit pas constitué d'acides aminés canoniques;
b. préparer à partir de la molécule organique de formule générale (I) un conjugué protéique de formule générale (II)
P-(L)ₚ -T-Z (II)
dans laquelle
P est une protéine rapporteuse,
T et Z ont la même définition qu'à l'étape a,
L est un peptide comprenant deux ou plusieurs acides aminés naturels et p vaut 0 ou 1 ;
c. incorporer le conjugué protéique obtenu à l'étape b) dans une lignée cellulaire ;
d. mesurer le renouvellement du conjugué protéique et le comparer avec le renouvellement d'une protéine témoin afin de déterminer si le conjugué protéique est marqué par un MAAD induisant une dégradation intracellulaire de la protéine,
e. générer un pool de cellules mutantes, chacune présentant un défaut dans un gène différent, et
f. incorporer le conjugué protéique marqué par le MAAD identifié à l'étape d) dans le pool de cellules mutantes généré à l'étape e) afin d'isoler les cellules mutantes qui ne parviennent pas à dégrader sélectivement le conjugué protéique, identifiant ainsi les facteurs intervenant dans la dégradation sélective du conjugué protéique marqué par le MAAD.

2. Procédé selon la revendication 1, dans lequel T est un groupement organique de formule générale (III)
-S-(Y)ₘ- (III)
dans laquelle S est un groupement organique de formule générale (IV) 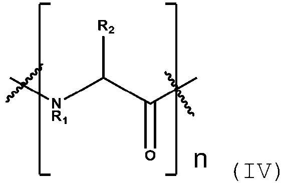
dans laquelle
R₁ représente un atome d'hydrogène, un radical alkyle en C₁ à C₁₀ linéaire ou ramifié, saturé ou insaturé, ou forme avec R₂ un système cyclique,
R₂ est choisi parmi le groupe constitué de l'hydrogène, d'un groupe alkyle substitué ou non substitué, d'un groupe alcényle substitué ou non substitué, d'un groupe alcynyle substitué ou non substitué, d'un groupe cycloalkyle substitué ou non substitué, d'un groupe hétérocycloalkyle substitué ou non substitué, d'un groupe aryle substitué ou non substitué, d'un groupe hétéroaryle substitué ou non substitué, d'un groupe aralkyle substitué ou non substitué, d'un groupe aralcényle substitué ou non substitué, d'un groupe hétéroaralkyle substitué ou non substitué et d'un groupe hétéroaralcényle substitué ou non substitué,
n vaut de 1 à 5,
Y est un acide aminé canonique ou un peptide comprenant deux acides aminés canoniques ou plus, et m vaut 0 ou 1.

3. Procédé selon la revendication 2, dans lequel n vaut 1 ou 2, de préférence 1.

4. Procédé selon la revendication 2 ou 3, dans lequel R₂ est choisi parmi le groupe constitué d'un groupe alkyle substitué, d'un groupe alcényle substitué ou non substitué, d'un groupe aryle substitué, d'un groupe hétéroaryle substitué ou non substitué, d'un groupe aralkyle substitué ou non substitué, d'un groupe aralcényle substitué ou non substitué, d'un groupe hétéroaralkyle substitué ou non substitué, et d'hétéroaralcényle substitué ou non substitué, de préférence d'alkyle substitué et d'aralkyle substitué.

5. Procédé selon l'une quelconque des revendications 2 à 4, dans lequel S est un acide aminé modifié.

6. Procédé selon l'une quelconque des revendications 2 à 5, dans lequel S comprend un acide aminé D non-canonique.

7. Procédé selon l'une quelconque des revendications 2 à 6, dans lequel S est un acide aminé modifié par voie enzymatique.

8. Procédé selon l'une quelconque des revendications 2 à 6, dans lequel S est un acide aminé modifié chimiquement.

9. Procédé selon l'une quelconque des revendications précédentes, dans lequel Z est choisi parmi le groupe constitué de NH₂, SH₂, OMe et OEt, de préférence NH₂ et OMe, et de manière encore plus préférentielle est NH₂.

10. Procédé selon la revendication 9, dans lequel S est un acide aminé canonique et m est égal à 0.

11. Procédé selon l'une quelconque des revendications 2 à 10, dans lequel S est choisi parmi le groupe constitué de
| | |
|---|---|
| 1 | 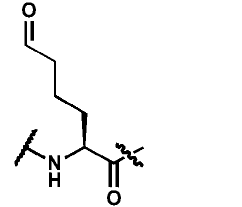 |
| 2 | 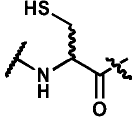 |
| 3 | 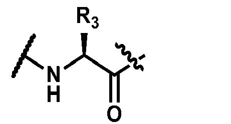 où R₃ est le résidu d'un acide aminé canonique |
| 4 | 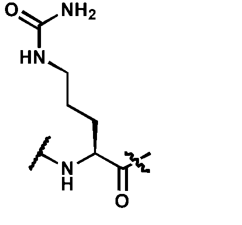 |
| 5 | 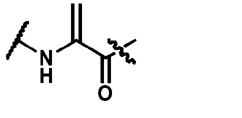 |
| 6 | 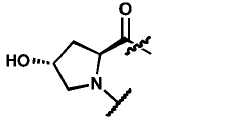 |
| 7 | 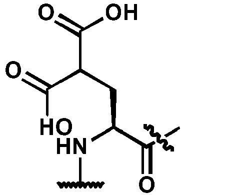 |
| 8 | 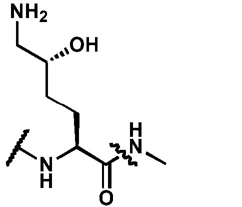 |
| 9 | 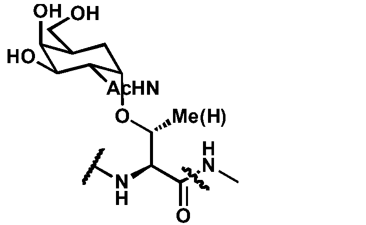 |
| 10 | 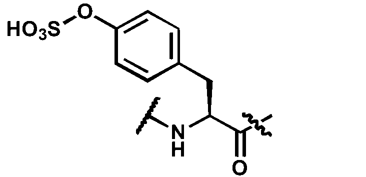 |
| 11 | 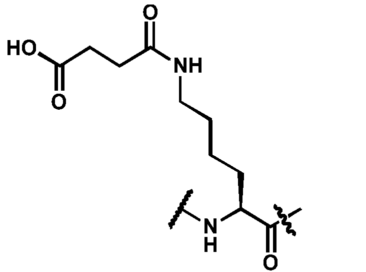 |
| 12 | 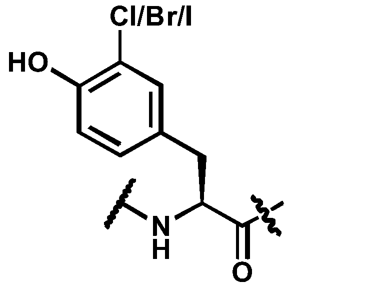 |
| 13 | 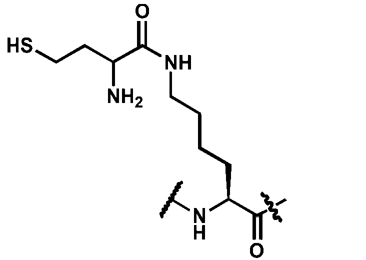 |
| 14 | 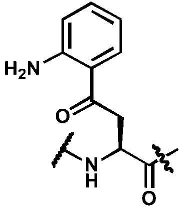 |
| 15 | 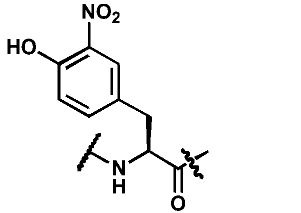 |
| 16 | 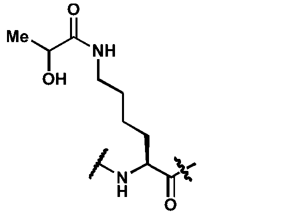 |
| 17 | 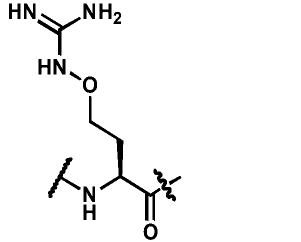 |
| 18 | 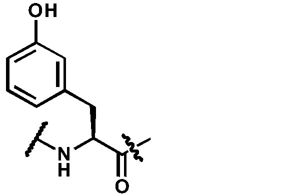 |
| 19 | 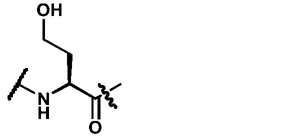 |
| 20 | 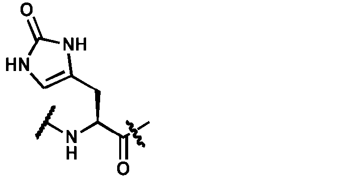 |
| 21 | 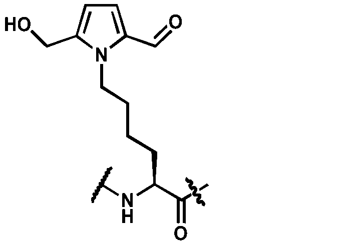 |
| 22 | 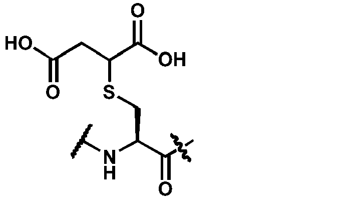 |
| 23 | 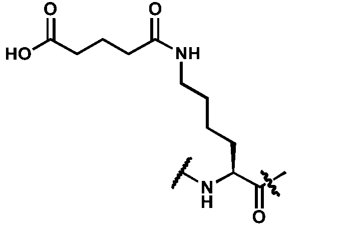 |

12. Procédé selon l'une quelconque des revendications précédentes, dans lequel la préparation du conjugué protéique de l'étape b) est réalisée par conjugaison chimio-enzymatique du composé de l'étape a) à la protéine rapporteuse, en particulier à l'aide de la sortase.

13. Procédé selon l'une quelconque des revendications précédentes, dans lequel la génération du pool de cellules mutantes selon l'étape e) est réalisée à l'aide d'un système de knock-out inductible basé sur CRISPR, en particulier à l'aide d'un système de knock-out dépendant de la doxycycline.
